# EUROPEAN PATENT APPLICATION

(11) **EP 4 631 449 A1**
(43) Date of publication of application: **15.10.2025**
(21) Application number: 23888091.8
(22) Date of filing: 09.11.2023
(51) Int. Cl.: A61B 18/00, A61B 18/12, A61B 18/14

(54) **ABLATION CATHETER**

(30) Priority: 10.11.2022 CN 202211420545
(71) Applicant: Hangzhou Dinova EP Technology Co., Ltd., Hangzhou, Zhejiang 311103 (CN)
(72) Inventor: LIU, Cheng, Hangzhou, Zhejiang 311103 (CN); LI, Hualin, Hangzhou, Zhejiang 311103 (CN); ZHANG, Sicheng, Hangzhou, Zhejiang 311103 (CN)
(74) Representative: Metida
(86) International application number: PCT/CN2023/130850
(87) International publication number: WO 2024/099406

(57) **Abstract**

This application discloses an ablation catheter (100, 300, 500, 600, 700, 800), including a catheter body (10) and a support framework (201, 202, 203, 204, 205) provided at a distal end of the catheter body (10). The support framework (201, 202, 203, 204, 205) includes a connecting frame (301, 302, 303, 304, 305) and a bearing frame (401, 402, 403, 404, 405) being provided distally relative to the connecting frame (301, 302, 303, 304, 305). A distal end of the connecting frame (301, 302, 303, 304, 305) is connected to a proximal end of the bearing frame (401, 402, 403, 404, 405), and a proximal end of the connecting frame (301, 302, 303, 304, 305) is connected to the distal end of the catheter body (10). A conducting portion (31, 32) is provided on the connecting frame (301, 302, 303, 304, 305), an electrode unit (41, 43, 45) insulated from the conducting portion (31, 32) is provided on the bearing frame (401, 402, 403, 404, 405), wherein an electrode area of the electrode unit (41, 43, 45) is less than that of the conducting portion (31, 32). The conducting portion (31, 32) can be connected to one pole of an energy generator, and the electrode unit (41, 43, 45) can be connected to another pole of the energy generator, so that ablation energy output by the energy generator is transferred to a target tissue region through the cooperation of the conducting portion (31, 32) and the electrode set (41, 43, 45).

## Description

This application claims the priority to Chinese Patent Application No. 202211420545.9, filed on Nov. 10, 2022 and entitled "Ablation Catheter", the entire contents of which are hereby incorporated by reference.

### TECHNICAL FIELD

This application relates to the technical field of medical devices, and in particular to an ablation catheter.

### DACKGROUND

Complex atrial fibrillation refers to atrial fibrillation that cannot be cured just by pulmonary vein isolation. Essentially, it belongs to persistent atrial fibrillation, triggered by other complex factors besides pulmonary veins. Therefore, due to its complex pathogenesis, the difficulty and complexity of treatment are increases, and postoperative recurrence is also prone to form recurrent atrial fibrillation. Patients with atrial fibrillation lasting more than 6 months have a 64% increased risk of postoperative recurrence, while patients with atrial fibrillation lasting more than 2 years still have a recurrence rate of over 50% even after multiple ablations. This is because as the duration of atrial fibrillation prolongs, the normal tissues of the atrium will gradually become fibrotic, which will affect the normal electrical conduction function. The longer the time, the less likely these fibrotic tissues are to be recovered.

The key to the success rate of persistent atrial fibrillation ablation lies in improving the integrity and transmurality of the ablation line. For persistent atrial fibrillation, the main sites of ablation treatment are the mitral valve isthmus, tricuspid valve isthmus, and ablation isolation of the left atrial apex line.

The current problem with ablation for the treatment of persistent atrial fibrillation lies in the contradiction between the ablation method and the ablation depth. When using the existing bipolar ablation method, the electric field is difficult to diffuse to deeper areas, often unable to form deeper damage. The ablation depth is not enough to form transmural damage. However, when using the existing unipolar ablation method, the electric field direction of the unipolar ablation method is from the electrode in the body to the negative pole on the surface of the body, so it is easy to reach deeper areas. However, the unipolar ablation method stimulates the ablation target greatly, which can easily cause damage to the aorta and esophagus.

### SUMMARY

In view of the above, an object of the present application is to provide an ablation catheter to solve the problem of insufficient ablation depth and excessive stimulation to the ablation object during the ablation process.

This application provides an ablation catheter including:
a catheter body;
a support framework being provided at a distal end of the catheter body, including a connecting frame and a bearing frame being arranged distally relative to the connecting frame, a distal end of the connecting frame being connected to a proximal end of the bearing frame, a proximal end of the connecting frame being connected to the distal end of the catheter body, a conducting portion being provided on the connecting frame, an electrode unit insulated from the conducting portion being provided on the bearing frame, an electrode area of the electrode unit being less smaller than that of the conducting portion,
wherein, the conducting portion is capable of connecting one pole of an energy generator, and the electrode unit is capable of connecting another pole of the energy generator, so that ablation energy output by the energy generator is transferred to a target tissue region through a cooperation of the conducting portion and the electrode unit.

The ablation catheter provided by embodiments of the application includes a catheter body and a support framework provided at a distal end of the catheter body. The support framework includes a connecting frame and a bearing frame being arranged distally relative to the connecting frame, a distal end of the connecting frame being connected to a proximal end of the bearing frame, a proximal end of the connecting frame being connected to the distal end of the catheter body, a conducting portion being provided on the connecting frame, an electrode unit insulated from the conducting portion being provided on the bearing frame, an electrode area of the electrode unit being less smaller than that of the conducting portion. The conducting portion is capable of connecting one pole of an energy generator, and the electrode unit is capable of connecting another pole of the energy generator, so that ablation energy output by the energy generator is transferred to a target tissue region through a cooperation of the conducting portion and the electrode unit.

On one hand, due to the conducting portion is located at the proximal end of the support framework and the electrode unit is located at the distal end of the support framework, positions of the conducting portion and the electrode unit are relatively dispersed compared to the adjacent two electrodes of the electrode unit on the support framework. Therefore, the coverage range of the electric field formed between the conducting portion and the electrode unit will be larger, allowing the electric field to diffuse to deeper areas to improve the depth of ablation damage. At the same time, due to the electrode area of the electrode unit is smaller than that of the conducting portion, the current density of the electrode unit is relatively high, resulting in more concentrated ablation energy in the bearing frame. In case of more concentrated ablation energy, the ablation depth of tissue ablation using the bearing frame will be deeper, and the ablation effect will be better. On the other hand, since both the conducting portion and the electrode unit are set on the support framework, compared to the existing ablation catheter where the positive electrode is set on the support framework and the negative electrode is an electrode plate attached to the skin of the human body, both the positive and negative electrodes of the present application are set on the support framework, thus most of the current between the positive and negative electrodes only exists at positions near the support framework and does not flow through the skeletal muscles in the back and other areas, thereby reducing the stimulation to the ablation subject's body.

### BRIEF DESCRIPTION OF DRAWINGS

In order to provide a clearer description of embodiments of the present application or technical solutions in the art, a brief description will be given to the accompanying drawings required for the embodiments or existing technology. It is obvious that the accompanying drawings described below are only some embodiments of the present application. For those skilled in the art, other drawings can be obtained based on these drawings without requiring creative labor.
Fig. 1 is a schematic view of an ablation catheter provided in a first embodiment of the present application.
Fig. 2 is an unfolded view of a part of a connecting frame of the ablation catheter of Fig. 1.
Fig. 3 is a schematic view of a bearing frame of the ablation catheter of Fig. 1.
Fig. 4 is a schematic view showing the ablation catheter of Fig. 1 performing surgery on the human heart.
Fig. 5 is a simulation diagram of an electric field distribution of the ablation catheter of Fig. 1.
Fig. 6 is a schematic view of a first discharge mode for segmented discharge of the ablation catheter of Fig. 1.
Fig. 7 is a schematic view of a second discharge mode for segmented discharge of the ablation catheter of Fig. 1.
Fig. 8 is a schematic view of a third discharge mode for segmented discharge of the ablation catheter of Fig. 1.
Fig. 9 is a schematic view of an ablation catheter provided in a second embodiment of the present application from a first perspective.
Fig. 10 is a schematic view of the ablation catheter of Fig. 9 from a second perspective.
Fig. 11 is a simulation diagram of an electric field distribution of the ablation catheter of Fig. 9.
Fig. 12 is a schematic view of a first discharge mode for segmented discharge of the ablation catheter of Fig. 9.
Fig. 13 is a schematic view of a second discharge mode for segmented discharge of the ablation catheter of Fig. 9.
Fig. 14 is a schematic view of a third discharge mode for segmented discharge of the ablation catheter of Fig. 9.
Fig. 15 is a schematic view of an ablation catheter provided in a third embodiment of the present application from a first perspective.
Fig. 16 is a schematic view of the ablation catheter of Fig. 15 from a second perspective.
Fig. 17 is a schematic view of the ablation catheter of Fig. 15 from a third perspective.
Fig. 18 is a schematic view of a connecting member of the ablation catheter of Fig. 15.
Fig. 19 is a schematic view of an ablation catheter provided in a fourth embodiment of the present application from a first perspective.
Fig. 20 is a schematic view of the ablation catheter of Fig. 19 from a second perspective.
Fig. 21 is a schematic view of an ablation catheter provided in a fifth embodiment of the present application.
Fig. 22 is a schematic view of an ablation catheter provided in a sixth embodiment of the present application.

Main reference numbers in the drawings:
ablation catheter -- 100, 300, 500, 600, 700, 800;
catheter body -- 10;
bendable section -- 11;
support section -- 12;
support framework -- 201, 202, 203, 204, 205;
equatorial position -- P1;
connecting frame -- 301, 302, 303, 304, 305;
first connecting end -- 3051;
second connecting end -- 3052;
mounting hole -- 306;
conducting portion -- 31, 32;
connecting rod -- 33, 34;
mesh structure -- 330, 340, 350;
mesh unit -- 36, 37, 37A;
main mesh -- 360, 370, 370A;
first main mesh -- 361, 371, 371A, 371B, 371C;
second main mesh -- 362, 372, 372A;
connecting mesh -- 380, 390, 390A;
first connecting mesh -- 381;
second connecting mesh -- 382;
first sub-mesh -- 391, 391A, 391B, 391C;
second sub-mesh -- 392, 392A, 392B, 392C;
third sub-mesh -- 393;
bearing frame -- 401, 402, 403, 404, 405;
electrode unit -- 41, 43, 45;
electrode -- 410, 430;
ablation electrode -- 451;
mapping electrode -- 452;
first electrode -- 411, 431, 431A;
first ring -- 412, 432;
second electrode -- 413, 433, 433A;
second ring -- 414, 434;
bearing rod -- 42, 44, 46, 47, 48, 49;
first bearing rod -- 421;
first sub-bearing rod -- 4211;
second sub-bearing rod -- 4212;
second bearing rod -- 422;
reverse portion-- 460;
concave area -- 4601;
bending portion -- 4602;
first rod -- 481;
second rod -- 482;
rod body -- 491;
insulating sleeve -- 492;
traction member -- 50, 50A;
reference electrode -- 61, 62;
microelectrode -- 63;
head electrode-- 65;
connecting member -- 70;
first connecting portion-- 71;
positioning hole -- 711;
second connecting portion-- 72;
first groove -- 721;
second groove -- 722;
delivery device -- 200;
sheath catheter -- 21.

The present application will be further described by the following specific embodiments in combination with the attached drawings.

### DESCRIPTION OF EMBODIMENTS

Technical solutions according to embodiments of the present application will be clearly and completely described with reference to the drawings in the embodiments of the present application. Apparently, the embodiments described are merely some embodiments, but not all of the embodiments of the present application. All other embodiments obtained by persons of ordinary skill in the art based on the embodiments of the present application without creative efforts shall fall within the protection scope of the present application.

Firstly, it should be noted that in the technical field of interventional medicine, along the delivery path of the device, an end of the device close to the operator is generally referred to as a "proximal end", and an end of the device away from the operator is referred to as a distal end. Specifically, for the delivery device used to deliver and release implantable devices into the patient's body, the distal end refers to the end where the delivery device can be freely inserted into the animal or human body, and the proximal end refers to the end where the delivery device is operated by the user or machine.

The term "axial **direction"** generally refers to a length direction of the medical device when it is delivered, the term "radial **direction"** generally refers to a direction of the medical device perpendicular to the "axial **direction",** and the term "circumferential direction" generally refers to a direction surrounding the "axial direction". Based on this principle, this disclosure defines the "axial direction", "radial direction", and "circumferential direction" of the relevant components of the ablation catheter. These definitions are only for the convenience of expression and do not constitute limitations on the present application. If there are exceptional circumstances, reasonable explanations can be made based on the attached drawings and the usual understanding of those skilled in the art.

It can be understood that the terms used in the specification, claims, and attached drawings of this application are only for describing specific embodiments and are not intended to limit the scope of this application. The terms "first", "second", and etc., in the specification and claims of this application, as well as in the attached drawings, are used to distinguish different objects and not to describe a specific order. Unless otherwise explicitly stated in the disclosure, singular forms of "a/an" and "the/said" are also intended to include plural forms. The term "including/**comprising**" and any variations thereof are intended to cover non-exclusive inclusions. In addition, the present application can be implemented in various forms and is not limited to the embodiments described in this embodiments. The purpose of providing the following specific embodiments is to facilitate a clearer and more thorough understanding of the disclosed content of this application, where words indicating directions such as up, down, left, and right, and etc., are only for the positions of the structures shown in the corresponding figures.

The subsequent description in the specification are preferred embodiments for implementing the present application, however, the above description is for the purpose of illustrating the general principles of the present application and is not intended to limit the scope of the present application. The protection scope of this application shall be as defined in the claims.

### Embodiment 1

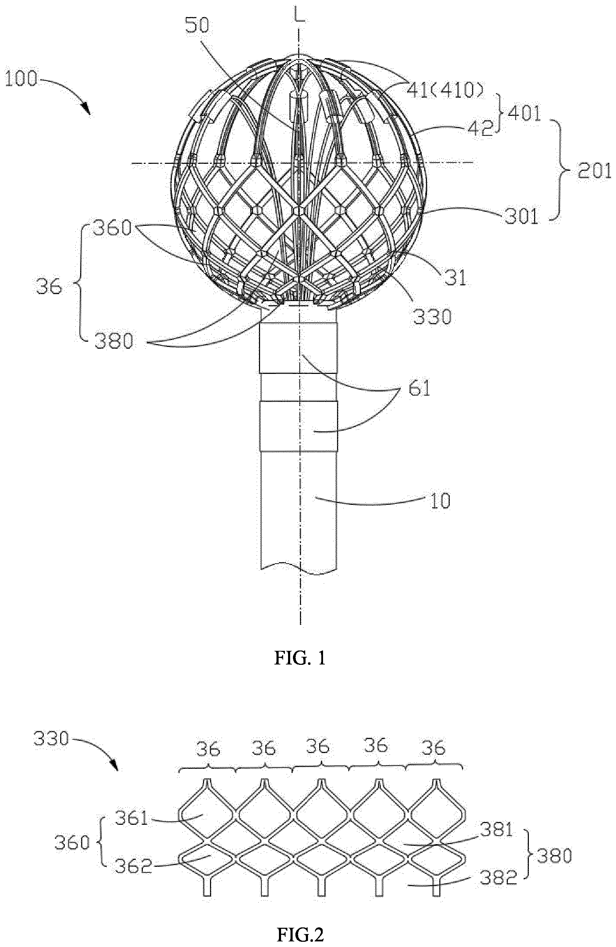

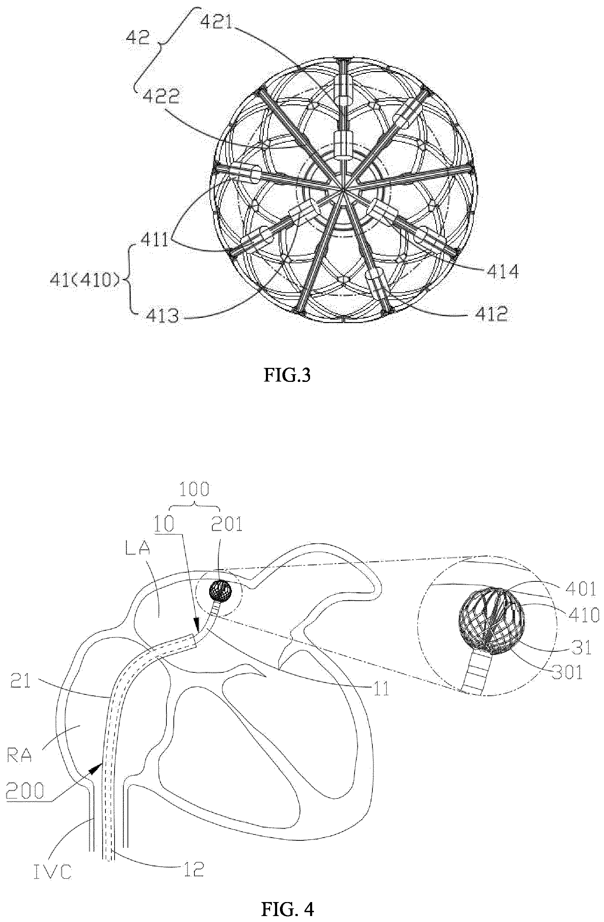

Please referring to Fig. 1 and Fig. 4, Fig. 1 shows a schematic view of an ablation catheter 100 provided in an embodiment of the present application, and Fig. 4 is a schematic view showing a scenario where the ablation catheter 100 of Fig. 1 performs surgery on the human heart. The ablation catheter 100 includes a catheter body 10 and a support framework 201, wherein the support framework 201 is provided at a distal end of the catheter body 10. The ablation catheter 100 may be delivered to an interior of the heart through a delivery device 200. The delivery device 200 includes but is not limited to a sheath catheter 21, a handle, and other components. It should be noted that a specific construction of the delivery device 200 may refer to relevant technologies in the art field, which will not be repeated in this application.

For those skilled in the art, it should be understand that Fig. 1 is only an example of the ablation catheter 100 and does not constitute a limitation on the ablation catheter 100. The ablation catheter 100 may include more or fewer components than that shown in Fig. 1, or may be combined with some components, or different components. For example, the ablation catheter 100 may further include temperature sensors, and etc. The temperature sensors are used to detect the temperature of the target tissue during the ablation process to prevent temperatures from being too low or too high.

The catheter body 10 is tubular-shaped. For example, in this embodiment, the catheter body 10 is configured as a hollow tubular structure. A central axis L of the catheter body 10 extends along a direction from a distal end to a proximal end. The catheter body 10 is configured with a single axial lumen or a central lumen (not shown in the drawings) therein. According to actual needs, the catheter body 10 may be configured with multiple lumens to accommodate wires, leads, sensor cables, and any other structures such as wires, cables, and/or tubes that may be required in specific applications.

It can be understood that the catheter body 10 may also be constructed in any suitable way and made of any suitable material, such as including an outer wall made of polymer materials such as polyurethane, polyether block amide (PeBax), and etc. The catheter body 10 has a certain degree of flexibility and can bend to adapt to the curved structure inside the heart. A diameter of the catheter body 10 is approximately 8F. It should be noted that the size of the catheter body 10 is only for illustration purposes and does not constitute a specific limitation.

In some embodiments, the catheter body 10 is configured as an adjustable bendable structure. A distal portion of the catheter body 10 is configured as an elastic structure. The catheter body 10 includes a bendable section 11 and a support section 12. A distal end of the bendable section 11 is connected to the support framework 201, and a proximal end of the bendable section 11 is connected to a distal end of the support section 12. A proximal end of support section 12 is connected to the handle. An elastic modulus of the bendable section 11 is less than that of the support section 12. On one hand, the support section 12 can provide support for the bendable section 11 to facilitate the transportation of the catheter body 10 to the target tissue area; on the other hand, it facilitates the retraction or release of the support framework 201 for the catheter body 10, and improves the ability of the ablation catheter 100 to adapt to anatomical shapes. The bendable section 11 is a soft section, being different from the support section 12 in the elastic modulus. The material of the bendable section 11 includes but is not limited to PeBax, thermoplastic polyurethane (Tpu), and etc. The hardness of PeBax may be 35D or 40D. The hardness of Tpu may be in range of 70A-110A. Specifically, the hardness of Tpu may be but not limited to 70A, 75A, 80A, 85A, 90A, 95A, 100A, 105A, 110A, and etc. For example, PeBax uses Pebax35D, and Tpu uses Tpu80A. For example, in this embodiment, the material of the support section 12 is PeBax, and the hardness of PeBax for the support section 12 may be in range of 40D-72D. For example, Pebax for the support section 12 is PeBax 72D. It should be noted that Pebax 35D refers to PeBax with a hardness of 35D, Tpu80A refers to Tpu with a hardness of 35A, and Pebax72D refers to PeBax with a hardness of 72D. The bendable section 11 and the support section 12 may be connected together through, but not limited to, hot melt method. In other embodiments, the catheter body 10 is configured as a rigid structure as a whole.

The support framework 201 can contract and expand radially. The proximal end of the support framework 201 is connected to the distal end of the catheter body 10, and the distal end of the support framework 201 converges towards the central axis L of the catheter body 10. The support framework 201 is constructed as an elastic mesh basket, so that a contacting surface formed by the outer surface of the support framework 201 is easy to be attached the target tissue area, thereby enabling the support framework 201 to be attached to and positioned at any angle on the target tissue area.

The support framework 201 has a contracted state and an expanded state. It should be noted that the contracted state refers to a state in which at least a part of the support framework skeleton 201 is not fully expanded, that is, the support framework 201 is constrained by a constraining element, for example, the support framework 201 is loaded into the sheath catheter 21 of the delivery device 200, and at this time, the support framework 201 contracts radially and the outer diameter of the support framework 201 is smaller than that of the sheath catheter 21. The expanded state refers to the fully expanded state of the support framework 201, that is, the support framework 201 is in a freely unfolded working state. For example, the support framework 201 is not pressed into the sheath catheter 21 and is not subjected to any other external force. At this time, the support framework 201 expands freely in the radial direction, and the outer diameter of the support framework 201 is larger than that of the sheath catheter 21. In this embodiment, Fig. 1 illustrates the support framework 201 in an expanded state. At this time, the two ends of the support framework 201 are folded and the middle of the support framework 201 is unfolded, and the profile of the support framework 201 is generally in the shape of a mesh basket. In the expanded state, the support framework 201 can also have other configurations, such as spherical shaped, egg shaped, pumpkin shaped, lantern shaped, elliptical shaped, and etc.

On the basis of the structure illustrated in Fig. 1, the support framework 201 can contract radially relative to the central axis L of the catheter body 10, that is, the middle of the support framework 201 also contracts towards the central axis L of the catheter body 10. In the contracted state, the support framework 201 will straighten out and roughly form a linear shape. Among them, the axial length of the support framework 201 in the contracted state will be greater than that in the expanded state.

In the contracted state, the support framework 201 can be accommodated within the sheath catheter 21 for easy delivery into the human body through the sheath catheter 21. When reaching the target ablation site through the sheath catheter 21, the support framework 201 is released from the sheath catheter 21 and then expands radially to the expanded state shown in Fig. 1. The radial expansion of the support framework 201 may be self-expansion when it extends out of the sheath catheter 21, or under human intervention after extending out of the sheath catheter 21.

The support framework 201 is made of materials with shape memory effect, which enables the support framework 201 to have good sheath performance. The support framework 201 can be prepared as a whole or divided into different local structures for preparation. For example, the support framework 201 can be formed by weaving woven wires with shape memory effect; or, pipes with shape memory effect as a whole can be cut to make them; or, the support framework 201 may also be obtained using 3D metal printing technology; or, a part of the support framework 201 can be formed by weaving woven wires with shape memory effect, while another part of the support framework 201 can be made by cutting pipes with shape memory effect, and then the two parts can be connected to each other fixedly through welding or connecting structures. Among them, materials with shape memory effect include but are not limited to metal materials and non-metallic materials with memory effect. Non-metallic materials are, for example, elastic polymer materials or other hyperelastic materials. Metal materials include, but are not limited to, biocompatible metal materials such as stainless steel, nickel titanium alloys, cobalt based alloys, etc. Optionally, the metal material is stainless steel. In this embodiment, the support framework 201 is formed by cutting and shaping a nickel titanium alloy tube.

In this embodiment, the support framework 201 includes a connecting frame 301 and a bearing frame 401. In the fully expanded state of the support framework 201, both the connecting frame 301 and the bearing frame 401 are frame structures with internal spaces. The bearing frame 401 is positioned at the distal relative to the connecting frame 301. The distal end of the connecting frame 301 is connected to the proximal end of the bearing frame 401, and the proximal end of the connecting frame 301 is connected to the distal end of the catheter body 10. The connecting frame 301 is provided with a conducting portion 31, and the bearing frame 401 is provided with an electrode unit 41 that is insulated from the conducting portion 31. An electrode area of the electrode unit 41 is smaller than that of the conducting portion 31. Among them, the conducting portion 31 may be connected to one pole of an energy generator, and the electrode unit 41 may be connected to the other pole of the energy generator to transfer the ablation energy of the energy generator to the target tissue area through the cooperation of the conducting portion 31 and the electrode unit 41.

For the ablation catheter 100 of the present application, on one hand, due to the conducting portion 31 being located at the proximal end of the support framework 201 and the electrode unit 41 being located at the distal end of the support framework 201, the positions of the conducting portion 31 and the electrode unit 41 are relatively dispersed compared to the positions of adjacent electrodes of the electrode unit 41 on the bearing frame 401. Therefore, the coverage range of the electric field formed between the conducting portion 31 and the electrode unit 41 will be larger, allowing the electric field to diffuse to deeper areas to improve the depth of ablation damage. At the same time, due to the electrode area of the electrode unit 41 being smaller than that of the conducting portion 31, the current density of the electrode unit 41 is relatively large, which makes the ablation energy more concentrated in the bearing frame 401. Thus, in case of more concentrated ablation energy, using the bearing frame 401 to adhere to the tissue will result in deeper ablation depth and better ablation effect. On the other hand, since the conducting portion 31 and the electrode unit 41 are both located on the support framework 201, compared to the existing ablation catheter 100 where the positive pole is on the support framework and the negative pole is an externally attached negative pole plate, both the positive and negative poles of the present application are located on the support framework 201. Therefore, most of the current between the positive and negative poles only exists near the support framework 201 and does not flow through the skeletal muscles of the back and other areas, thereby reducing the stimulation of the ablation subject's body.

It should be noted that the term "electrode area" refers to the effective current transmission area of the electrode unit 41 or the conducting portion 31, that is, the electrode area can support the flow of current through the electrode, for example, between the electrode and the target point in contact with the electrode.

Optionally, the axial length of the connecting frame 301 is equal to the axial length of the bearing frame 401, ensuring that the connecting frame 301 provides a certain support and fixation to the bearing frame 401, while also ensuring that the electrode unit 41 has sufficient space to be placed on the bearing frame 401. In some embodiments, the axial length of the connecting frame 301 may also be less than or greater than the axial length of the bearing frame 401. For example, the axial length of the connecting frame 301 is greater than that of the bearing frame 401. Therefore, compared to the axial length of the connecting frame 301 being equal to that of the bearing frame 401, this further increases the electrode area of the conducting portion 31 and enhances the support of the connecting frame 301, thereby ensuring stronger stability of the bearing frame 401 installed on the connecting frame 301.

In this embodiment, the bearing frame 401 includes multiple bearing rods 42. It can be understood that, on the one hand, the bearing rod 42 is used to provide radial support force for the ablation catheter 100, so as to avoid deformation of the bearing frame 401 and the occurrence of insufficient spacing between adjacent electrodes of the electrode unit 41 on the bearing frame 401, which may cause arcing or electric sparks and cause breakdown damage to the target tissue; on the other hand, the bearing rod 42 has good flexibility, thereby enhancing the flexibility of the bearing frame 401 and ensuring that the support framework 201 can adhere well to the target tissue area. The multiple bearing rods 42 are arranged at intervals around the central axis L of the support framework 201. The distal ends of the multiple bearing rods 42 are close to the central axis L of the support framework 201 and fixed to each other. The proximal ends of the multiple bearing rods 42 are connected to the distal end of the connecting frame 301, so that the proximal ends of the multiple bearing rods 42 are fixed through the connecting frame 301, thereby restricting the relative movement between any two bearing rods 42, and enabling the electrode unit 41 to be more stably installed on multiple bearing rods 42 to achieve stable ablation during operation. The distal ends of the multiple bearing rods 42 converge towards the central axis L of the guide tube body 10. The distal ends of the multiple bearing rods 42 may be connected to each other; or, the distal ends of the multiple bearing rods 42 may be disconnected from each other; or, some of the distal ends of the multiple bearing rods 42 are connected to each other and some of the distal ends of the multiple bearing rods 42 are disconnected from each other.

The connecting frame 301 includes a plurality of connecting rods 33 that are connected end-to-end to form a mesh structure 330, which helps to improve the support performance of the support framework 201. The proximal end of mesh structure 330 is connected to the distal end of catheter body 10, and the distal end of mesh structure 330 is connected to the proximal ends of the multiple bearing rods 42.

In one embodiment, please referring to Fig. 1 and Fig. 2, Fig. 2 shows an unfolded view of a part of the connecting frame 301 of the ablation catheter 100. The mesh structure 330 includes a plurality of mesh units 36 and a plurality of connecting meshes 380. In this embodiment, the mesh structure 330 as a whole serves as the conducting portion 31 to achieve a larger electrode area of the conducting portion 31 than the electrode area of the electrode unit 41. The plurality of mesh units 36 are arranged circumferentially around the central axis L of the support framework 201. At least one connecting mesh 380 is set between two adjacent mesh units 36. Each mesh unit 36 includes at least one main mesh 360. The at least one connecting mesh 380 shares one side with each of at least two main meshes 360 of two adjacent mesh units 36, respectively. The profiles of the main mesh 360 and the connecting mesh 380 are both polygons, such as diamonds, trapezoids, pentagons, and hexagons. In this embodiment, the profiles of the main mesh 360 and the connecting mesh 380 are both diamond shaped.

In this embodiment, each mesh unit 36 includes two main meshes 360, namely first main mesh 361 and second main mesh 362, respectively. The first main mesh 361 and the second main mesh 362 share one mesh node, adjacent first main meshes 361 share one mesh node, and adjacent second main meshes 362 share one mesh node. There are a first connecting mesh 381 and a second connecting mesh 382 set between two adjacent mesh units 36. The first connecting mesh 381 shares one side with each of two first main meshes 361 and two second main meshes 362 of two adjacent mesh units 36, respectively. The second connecting mesh 382 shares one side with each of two second main meshes 362 of two adjacent mesh units 36, respectively. Two adjacent second connecting meshes 382 share one side with each other, resulting in a smaller space occupied by the connecting frame 301, easier contraction of connecting frame 301, and stronger stability of edge to edge connections between adjacent meshes. The second connecting mesh 382 is an open polygon, with its proximal end being connected to the distal end of the catheter body 10. The connection manner between the proximal end of the second connecting mesh 382 and the distal end of the catheter body 10 includes but is not limited to one or a combination of welding, clamping, bonding, thermal fusion, threaded connection, sealing connection, etc. The specific connection manner may be selected according to the usage requirements or functional requirements.

In this embodiment, the second connecting mesh 382 is an open pentagon. The second connecting mesh 382 includes a proximal edge connected to the second main mesh 362, and is connected to the distal end of the catheter body 10 through the proximal edge. The distal end of the first main mesh 361 is connected to the proximal ends of multiple bearing rods 42, thereby restricting each bearing rod 42 from moving relative to the support framework 201. The connection manner between the distal end of the first main mesh 361 and the proximal ends of the multiple bearing rods 42 includes but is not limited to one or a combination of welding, clamping, bonding, thermal fusion, threaded connection, sealing connection, etc. The specific connection manner may be selected according to usage requirements or functional requirements. Specifically, the number of first main meshes 361 corresponds to the number of the bearing rods 42, and each first main mesh 361 is connected to a corresponding bearing rod 42. Optionally, the mesh node of the first main mesh 361 is connected to the bearing rod 42, thereby avoiding the mesh node of the connecting frame 301 from scratching the target tissue during the surgical process, enabling the connecting frame 301 to support and fix the multiple bearing rods 42 more evenly and stably, and improving the flexibility of the support framework 201. In some embodiments, the number of first main meshes 361 may be greater than the number of the bearing rods 42. In this embodiment, the connecting frame 301 includes thirty-six meshes, all of which are diamond shaped. It can be understood that the specific shape and quantity of the meshes are not limited in this application. For example, the shape and quantity of the meshes may be set according to actual needs.

Please referring to Fig. 1 and Fig. 3, Fig. 3 is a schematic view of the bearing frame 401 of the ablation catheter 100 of Fig. 1. Multiple bearing rods 42 are enclosed to form the mesh basket structure that can contract and expand radially. The number of the bearing rods 42 may be four, five, six, seven, eight, nine, ten, eleven, twelve, or any other suitable number. Optionally, the multiple bearing rods 42 are uniformly arranged along the circumference of the bearing frame 401. In other embodiments, the multiple bearing rods 42 may be unevenly spaced along the circumference of the bearing frame 401. In this embodiment, there are nine bearing rods 42 arranged circumferentially around the central axis L of the catheter body 10.

In this embodiment, the connecting frame 301 as a whole is configured as the conducting portion 31. In other embodiments, a part of the connecting frame 301 is configured as the conducting portion 31, for example, the distal end, proximal end, or middle portion of the connecting frame 301 is configured as the conducting portion 31. The electrode unit 41 includes a plurality of the electrodes 410 arranged on the bearing rods 42, wherein the electrodes 410 are spaced apart from the conducting portion 31. In this way, after the radial expansion of the support framework 201, each bearing rod 42 can attach to the inner wall of the tissue inside the heart, and the multiple electrodes 410 on the bearing rods 42 use the ablation energy output by the energy generator to perform tissue ablation.

Optionally, the multiple electrodes 410 form at least one ring in the circumferential direction of the bearing frame 401, with the plane on which the ring is located being perpendicular to the central axis L of the catheter body 10. This allows for more uniform ablation of the target tissue area by the support framework 201 when using the electrodes 410 to ablate the target tissue area, and makes it easier for the multiple electrodes 410 of the support framework 201 to map the potential when using the electrodes 410 to map the target tissue area.

In this embodiment, the multiple bearing rods 42 include multiple first bearing rods 421 and multiple second bearing rods 422. The electrodes 410 are all set on the first bearing rods 421, which means that not all bearing rods 42 are provided with electrodes 410. This is to provide more space for retracting the support framework 201 to the sheath catheter 21, making it easier to retract support framework 201into the sheath catheter 21.

Optionally, the multiple electrodes 410 include multiple first electrodes 411 and multiple second electrodes 413. Each first bearing rod 421 is provided with the first electrode 411. The multiple first electrodes 411 form a first ring 412 in the circumferential direction of the support framework 401. The multiple second electrodes 413 are evenly spaced in the circumferential direction on some of the first bearing rods 421, and form a second ring 414 in the circumferential direction of the support framework 401. The first ring 412 is relatively close to the proximal end of the support framework 401, and the second ring 414 is relatively close to the distal end of the support framework 401. The plane on which the first ring 412 is located and the plane on which the second ring 414 is located both are perpendicular to the central axis L of the catheter body 10. The plane on which the first ring 412 is located and the plane on which the second ring 414 is located are parallel. Optionally, the number of the electrodes 410 on the first ring 412 is less than the number of the electrodes 410 on the second ring 414, thereby facilitating the retracting/extending of the support framework 201 into/from the sheath catheter 21.

Specifically, in this embodiment, the multiple bearing rods 42 include three bearing rod groups, each of which includes two first bearing rods 421 and one second bearing rod 422. The arrangement of the electrodes 410 on the bearing rods 42 of each bearing rod group is as follows: the second bearing rod 422 does not have electrodes 410, and the number of the electrodes 410 set on the two first bearing rods 421 is not the same. For example, one of the first bearing rods 421 has two electrodes 410 thereon, and the other first bearing rod 421 has one electrode 410 thereon, so that the bearing frame 401 includes six proximal electrodes (i.e., the first electrodes 411) and three distal electrodes (i.e., the second electrodes 413). The six proximal electrodes also form a first ring 412 in the circumferential direction of the support framework 201. The plane on which the six proximal electrodes of the first ring 412 are located is also perpendicular to the central axis L of the catheter body 10. The three distal electrodes form a second ring 414 in the circumferential direction of the support framework 201, and the plane on which the three distal electrodes of the second ring 414 are located is perpendicular to the central axis L of the catheter body 10. In some embodiments, the multiple electrodes 410 may further include multiple third electrodes, ..., and multiple Nth electrodes, where "N" is an integer greater than 3. The multiple third electrodes form a third ring in the circumferential direction of the bearing frame 401, and the multiple Nth electrodes form an Nth ring in the circumferential direction of the bearing frame 401. The number of the rings of the bearing frame 401 is for illustration purposes only and does not constitute a specific limitation. The radial dimensions of the multiple rings gradually increase from the distal end to the proximal end of the support framework 401. The number of the electrodes 410 of the multiple rings gradually increases from the distal end towards the proximal end of the bearing frame 401.

Optionally, the radial size of the bearing frame 401 gradually increases from its distal end towards its proximal end, ensuring that the distance between the multiple electrodes 410 is within a suitable range to avoid arcing, while also ensuring that the electrodes 410 can better adapt to the target tissue area and maintain good adhesion. Among them, the first electrodes 411 are located close the proximal end of the bearing frame 401.

Optionally, the bearings 42 are symmetrically arranged around the central axis L of the support framework 201, ensuring that after the support framework 201 rotates a predetermined angle, uniform ablation of the target tissue area can be achieved. In some embodiments, the bearing rods 42 are asymmetrically arranged around the central axis L of the support framework 201.

The electrode 410 is made of platinum iridium alloy, gold alloy, stainless steel, nickel titanium, or any other biocompatible medical metal. The electrode 410 can be an annular electrode, a sheet electrode, a point electrode, or a spherical electrode, etc., which is not limited in this embodiment. Optionally, the cross-sectional shape of electrode 410 is flat, making it convenient for the support framework 201 to be retracted into or extend from the sheath catheter 21. In this embodiment, the electrode 410 is a flat electrode that surrounds the outer wall of bearing rod 42. The flat electrode is mounted around the bearing rod 42. The inner wall of the flat electrode is attached to the outer circumference of the bearing rod 42.

Each electrode 410 has a wire configured with an insulating layer welded to its inner wall. The bearing rod 42 includes a rod body and an insulating sleeve mounted around the rod body, wherein the material of the insulating sleeve is PEBAX or other polymer insulating material. The insulating sleeve may be configured as one layer, two layers, or multiple layers, which is not limited here. The cross-sectional shape of the rod body may be elliptical, circular, rectangular, semi-circular, shuttle shaped, etc., which is not limited here. In this embodiment, the rod is made by cutting and heat setting a nickel titanium tube, which gives the rod body excellent elastic properties and strength to be able to attach well to the target tissue. It can be understood that the rod body can also be made of other materials, such as stainless steel or polymer materials. The electrode 410 is mounted around the insulating sleeve to ensure insulation between the electrode 410 and the rod body.

Each electrode 410 on the bearing rod 42 is connected to the energy generator outside the ablation catheter 100 through the wire. Specifically, the wire is placed between the rod body and the insulating sleeve. That is, the wire with an insulating layer is welded to the inner wall of each electrode 410. One end of the wire is welded to a connector on the handle to connect to the energy generator, and the other end is connected to the inner wall of the electrode 410 through the lumen of the catheter body 10 and the internal space of the support framework 201. The electrode 410 can be connected to the wire not only through welding, but also through other special processes. The wire can be enameled wire. It can be understood that enameled wire has insulation properties and can withstand voltage greater than or equal to 500V. After the electrodes 410 are connected to the energy generator through the wires, the energy generator can provide electrical pulses to the electrodes 410 to perform electroporation surgery. The energy generator can supply multiple different waveforms or shapes of pulses to the electrodes 410 to achieve electroporation ablation of lesion tissue, such as sinusoidal AC pulses, DC pulses, square wave pulses, exponentially attenuated waveforms, etc., as well as combined AC/DC pulses or DC offset signals. The energy pulse train received by the electrodes 410 includes single-phase pulses or two-phase pulses, which can be configured with different parameters such as voltage, pulse width, repetition frequency, duty cycle, and number of pulses. The conducting portion 31 of the connecting frame 301 has conductivity, and the proximal end of the connecting frame 301 is connected to the distal end of the wire by welding. The proximal end of the wire is welded to the connector on the handle to connect the conducting portion 31 to the energy generator.

When the ablation catheter 100 of the first embodiment is used to transfer ablation energy to the target tissue area, it can be achieved through the cooperation of multiple electrodes 410 on the bearing frame 401 and the conducting portion 31 of the connecting frame 301. Specifically, the multiple electrodes 410 are connected to the positive pole of the energy generator, and the conducting portion 31 of the connecting frame 301 is connected to the negative pole of the energy generator. Alternatively, the multiple electrodes 410 can be connected to the negative pole of the energy generator, and the conducting portion 31 of the connecting frame 301 can be connected to the negative pole of the energy generator. A pulsed electric field is formed between the positive and negative poles to transfer ablation energy to the target tissue area.

In some embodiments, some of the electrodes 410 can be selected to cooperate with the conducting portion 31 of the connecting frame 301 to achieve ablation energy transfer. Specifically, some of the multiple electrodes 410 are connected to the positive pole of the energy generator, and the conducting portion 31 of the connecting frame 301 is connected to the negative pole of the energy generator. Alternatively, some of the multiple electrodes 410 can be connected to the negative pole of the energy generator, and the conducting portion 31 of the connecting frame 301 can be connected to the positive pole of the energy generator.

In some embodiments, only the multiple electrodes 410 on the bearing frame 401 can be selected to transfer ablation energy to the target tissue area, that is, at least one of the multiple electrodes 410 is connected to the positive pole of the energy generator, and at least one of the remaining electrodes 410 is connected to the negative pole of the energy generator. Alternatively, at least one of the multiple electrodes 410 is connected to the negative pole of the energy generator, and at least one of the remaining electrodes 410 is connected to the positive pole of the energy generator.

In some embodiments, at least one of the multiple electrodes 410 can be selected to be connected to one pole of the energy generator, and the other pole of the energy generator can be connected to a patch electrode attached to the patient's body surface. Alternatively, the conducting portion 31 of the connecting frame 301 can be connected to one pole of the energy generator, and the other pole of the energy generator can be connected to the patch electrode attached to the patient's body surface.

It can be understood that the high-voltage pulse can be selected as the energy form transmitted by at least one of the multiple electrodes 410 and the conducting portion 31, and other forms of ablation energy can also be additionally or alternatively transmitted, such as radiofrequency energy or any other suitable form of energy.

In some embodiments, the ablation catheter 100 further includes a reference electrode 61, which is used for electrocardiogram signal mapping to obtain reference potential. The reference electrode 61 is set on the catheter wall of the distal end of the catheter body 10. The connection manner between the reference electrode 61 and the catheter body 10 includes but is not limited to welding, crimping, bonding, integrated molding, and etc. The number of the reference electrode 61 includes one or multiple. Optionally, multiple reference electrodes 61 are arranged at intervals along the central axis L of the catheter body 10. In this embodiment, the reference electrode 61 includes two annular electrodes. It can be understood that the shape and quantity of the reference electrode 61 are not limited in this embodiment. The reference electrode 61 may be made of metal materials such as platinum, iridium, gold, silver, etc. Optionally, the axial length of the reference electrode 61 is approximately 1.2mm. It should be noted that the size of the reference electrode 61 is only for illustration purposes and does not constitute a specific limitation. There is an opening for extending of the wire is defined in the catheter body 10 at a position corresponding to the reference electrode 61. Each reference electrode 61 has a wire configured with an insulating layer welded to its inner wall. One end of the wire is welded to the connector of the handle, and the other end extends along the lumen of the catheter body 10 and through the opening to connect the inner wall of the reference electrode 61.

The first function of the reference electrode 61 on the catheter body 10 is to serve as the reference electrode, which is used for mapping electrocardiogram signals to obtain reference potentials. Specifically, the multiple electrodes 410 on the bearing frame 401 can be in good contact with the target tissue area, serving as mapping electrodes to map the electrocardiogram signals of the target tissue area and obtain potentials. The reference electrode 61 on the catheter body 10, although not in contact with myocardial tissue, can also obtain a reference potential from the fluid in contact with the reference electrode 61. Based on the difference between the reference potential obtained by the reference electrode 61 and the potential obtained by the electrode 410, a more accurate electrocardiogram signal can be obtained, thereby improving the accuracy of mapping. The second function of the reference electrode 61 on the catheter body 10 is to serve as a positioning electrode for the 3D mapping system, which is used to display the body of the catheter body 10 during the 3D modeling process. The third function of the reference electrode 61 on the catheter body 10 is to act as an ablation electrode, that is, the reference electrode 61 can be used as a negative pole to increase the discharge area of the negative pole and reduce the occurrence of electric sparks. For example, it can be understood that increasing the negative pole area may lead to a decrease in impedance, but this is a normal phenomenon and requires resetting the baseline impedance reference.

In this embodiment, the support framework 201 is a whole is configured as a cage-like structure. When the conducting portion 31 of the connecting frame 301 and the multiple electrodes 410 on the bearing frame 401 discharge, it can be regarded as local point ablation. By connecting them point by point, it is possible to achieve linear ablation of the tricuspid valve isthmus, the top line of the left atrium (LA), the bottom line of the right atrium (RA), the mitral valve isthmus, and other surgical requirements for arrhythmia treatment. Below is an explanation of the surgical procedure for the ablation catheter 100 in this application.

Please referring to Figs. 1-4, a brief explanation of the operation steps for performing surgery on the human heart using the ablation catheter 100 of the present application is as follows. By femoral vein puncture, the delivery device 200 is delivered through the inferior vena cava (IVC) and the right atrium to the left atrium. The ablation catheter 100 is released into the left atrium after bending through the delivery device 200. The abnormal lesion area is identified through three-dimensional mapping, and the top of the left atrium or mitral valve isthmus is isolated, as well as the removal of abnormal activation points. At this time, the electrodes 410 of the bearing frame 401 abut the target tissue area (such as abnormal myocardial tissue). When the electrodes 410 come into contact with the target tissue area, the conducting portion 31 of the connecting frame 301 and the multiple electrodes 410 on the bearing frame 401 cooperate to form a pulsed electric field for ablation, causing tissue necrosis in the target tissue area. The remaining electrodes 410 that do not come into contact with the target tissue area choose to turn off and not discharge. After ablation, the delivery device 200 and ablation catheter 100 are withdrawn from the left atrium and reach the right atrium, and the tricuspid valve isthmus is isolated. At this time, a pulse ablation electric field is also formed by the cooperation of the conducting portion 31 of the connecting frame 301 and the multiple electrodes 410 on the bearing frame 401 for ablation.

In the process of pulse ablation using the ablation catheter 100, a pulsed electric field is formed between the positive and negative poles to act on the target tissue area. In this embodiment, the connecting frame 301 formed by the mesh structures 330 is used as the negative pole, and the multiple electrodes 410 set on the bearing frame 401 act as the positive pole. On one hand, the positions of the positive and negative poles are relatively dispersed, which can achieve a larger electric field coverage range between the positive and negative poles, thereby improving the depth of ablation damage. On the other hand, compared to the electrode area of the electrodes 410 on the bearing frame 401 participating in the discharge, the entire connecting frame 301 acts as the conducting portion 31 participating in the discharge. The electrode area of the conducting portion 31 is larger than that of the electrodes 410, so the current density of the conducting portion 31 is relatively smaller. The current density of the electrode 410 on the bearing frame 401 is relatively larger, so the energy will be more concentrated in the bearing frame 401. The electrodes 410 of the bearing frame 401 mainly attach to the target tissue area for ablation. Therefore, in the case of more concentrated energy, the ablation depth using the bearing frame 401 to attach to the tissue will be deeper.

The ablation catheter 100 provided in this embodiment adopts a novel skeleton structure and bipolar ablation method, which utilizes the mesh structure 330 of the connecting frame 301 with a relatively small mesh area and the multiple electrodes 410 on the bearing frame 401 with a relatively large mesh area as positive and negative poles for ablation, not only fully utilizing the advantages of bipolar ablation method, which has less stimulation on the body and better ablation effect, but also greatly improving the ablation depth and ensuring ablation transparency, thereby improving the ablation effect of the ablation catheter 100.

Here is an explanation of 3D modeling and mapping. It can be understood that the electrodes 410 on the support framework 401 of the support framework 201 can be used not only for ablation, but also as a mapping electrode for mapping cardiac electrical signals. The electrode 410 is connected to the connector on the handle through wires, and then connected to the energy generator, multi-channel recorder, and 3D mapping system through cables. These three connections are synchronized, and the signal connected to the energy generator can also be connected to other systems through the output port for use. The electrocardiogram signal connected to the 3D mapping system is processed by a computer to generate an excitation map. Each mapping electrode is a single pathway. When the cardiac electrical signal passes through the mapping electrode, an electrocardiogram is generated, called a unipolar electrocardiogram (referred to as A), and another mapping electrode generates a unipolar electrocardiogram B. The actual use is a bipolar electrocardiogram, which uses A-B to generate electrocardiogram C to describe the distance between the electrocardiogram signal and these two mapping electrodes. The closer the distance to the mapping electrode, the larger the mapping signal, and the farther away the signal, the smaller the signal.

When the electrode 410 is connected to the 3D mapping system, the system forms an electrical circuit with each mapping electrode, and measures the field strength of each circuit. Through the relationship between the electric field gradient equation and the distance of the reference electrode 61, the mapping electrodes in contact with the inner wall of the heart are positioned and described. By moving the ablation catheter 100, the entire heart model can be constructed, and the cardiac electrical signals are recorded when contact, thus forming a heart model with excitation signals and completing the 3D modeling of the heart. Specifically, the ablation catheter 100 provided in this embodiment can be modeled in three dimensions using electric field modeling and mapping signals, and can also be corrected using computer tomography (CT) image data and digital subtraction angiography (DSA) image data to further improve model accuracy and reliability. Specifically, six specialized surface patches are affixed to the patient's chest, back, left and right armpits, neck, and thighs. Micro currents are distributed to the six patches through a three-dimensional mapping system, forming three mutually orthogonal electric fields within the patient's body. The electrodes 410 on the support framework 401 of the support framework of the ablation catheter 100 are located within these electric fields, forming circuits with the internal circuit of the three-dimensional mapping system in three different electric field directions, while detecting different field strengths in the three directions. By using the attenuation equation of field strength with distance, the distances of different electrodes 410 in various directions of the electric field can be obtained. Furthermore, the coordinates of each electrode 410 in the electric field can be obtained, and the ablation catheter 100 can be moved inside the heart or against the endocardial surface to collect the scanned points, thus obtaining the approximate heart cavity model. By using imaging techniques such as respiratory compensation and contact detection, the stability and accuracy of the electrode 410 sampling points can be further corrected, improving model accuracy. In addition, CT, DSA and other imaging data can be combined with algorithms to make more detailed corrections to the obtained model, improving the reliability and precision of 3D images and enhancing the reliability of the 3D model. The operator can accurately detect lesions under 3D guidance and deliver the ablation catheter 100 to the target position for ablation, thereby reducing the threshold for using the ablation catheter 100, shortening the operator's learning curve, and improving the user experience and expected success rate.

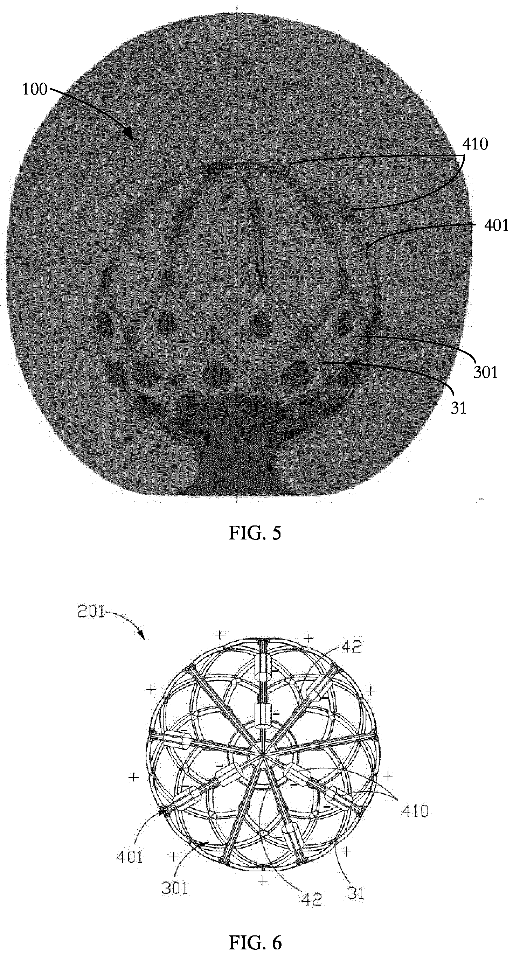

According to relevant literature research, the depth of the endocardial and epicardial muscle sleeves in the **mitral valve isthmus of the heart is 8.4 ± 2.7mm.** In this application, 6mm is used for calculation. The muscle sleeves are located in the coronary vein, thus the coverage width (ablation depth) of the pulsed electric field needs to be greater than the depth of the endocardial and epicardial muscle sleeves in the mitral valve isthmus to maintain complete damage and maintain therapeutic effect. Please referring to Figs. 1-5, Fig. 5 is a simulation diagram of the electric field distribution of the ablation catheter 100. The conducting portion 31 of the connecting frame 301 is the positive pole, and the electrodes 410 of the bearing frame 401 are the negative pole. The positive and negative poles of the ablation catheter 100 form a pulsed electric field as shown in Fig. 5 to transfer ablation energy to the target tissue area. When the electric field strength is 500V/cm, the maximum coverage width of the pulsed electric field between the conducting portion 31 of the connecting frame 301 and the electrodes 410 of the bearing frame 401 is about 6.5mm, which is greater than 6mm. The ablation depth exceeds 6mm, thus the mitral valve isthmus can be ablated through the wall to achieve therapeutic effect.

Optionally, in this embodiment, the ablation catheter 100 can also perform partition ablation on the target tissue area, thereby achieving precise partition ablation, and avoiding damage to the unexpectedly ablated tissue through partition local ablation. In addition, due to the targeted effect of ablation energy on the target tissue that needs to be ablated, the utilization rate of ablation energy is increased, the dissipation of ablation energy in the blood is reduced, and unnecessary bubbles generated by electrolyzing blood are reduced.

Please referring to Fig. 1 and Fig. 6, Fig. 6 is a schematic view of a first discharge mode for segmented discharge of the ablation catheter 100 of Fig. 1. In this embodiment, when only some the bearing rods 42 are in contact with the target tissue area, the conducting portion 31 and at least one electrode 410 of the some of the bearing rods 42 are conducted to transfer ablation energy to the target tissue area, with the at least one electrode 410 having a polarity opposite to that of the conducting portion 31.

Specifically, please referring to Fig. 6, the conducting portion 31 acts as the positive pole and the electrode 410 of the bearing frame 401 acts as the negative pole; Of course, in other embodiments, the conducting portion 31 can also be chosen as the negative pole, and the electrode 410 of the bearing frame 401 can be chosen as the positive pole. By detecting the impedance of each electrode 410, the attachment situation is determined. When only some of the bearing rods 42 is in contact with the target tissue area, at least one electrode 410 on the some of the bearing rods 42 in contact with the target tissue area and the conducting portion 31 are selected to participate in segmented discharge. For example, at least one electrode 410 on one of the some of the bearing rods 42 in contact with the target tissue area and the conducting portion 31 are selected to participate in segmented discharge. For another example, the electrodes 410 on several of the some of the bearing rods 42 in contact with the target tissue area and the conducting portion 31 are selected to participate in segmented discharge. Optionally, the electrodes 410 on two adjacent bearing rods 42 and the conducting portions 31 are selected to participate in segmented discharge to transfer ablation energy to the target tissue area. Among them, the polarity of the conducting portion 31 is opposite to that of the selected electrodes 410. After the ablation is completed, all electrodes 410 on the connecting frame 301 participate in the mapping.

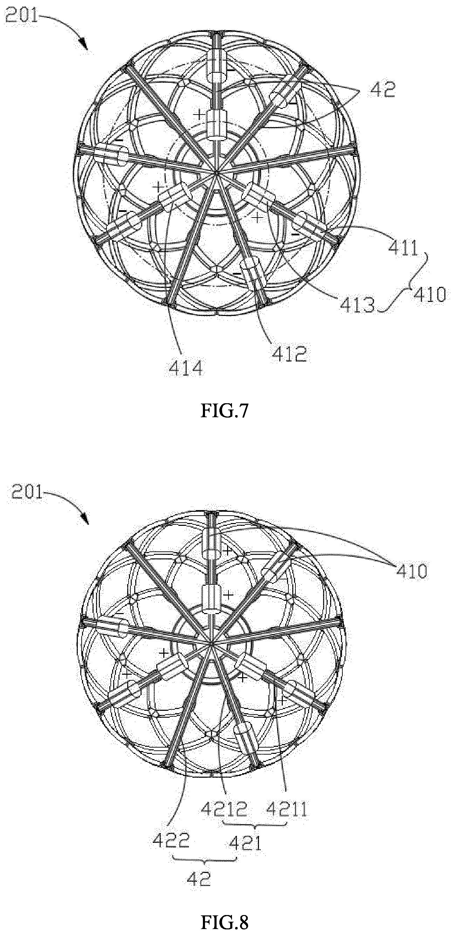

Please referring to Fig. 1, Fig. 7, and Fig. 8, Fig. 7 is a schematic view of a second discharge mode for segmented discharge of the ablation catheter 100 of Fig. 1, and Fig. 8 is a schematic view of a third discharge mode for segmented discharge of the ablation catheter 100 of Fig. 1. When only some of the bearing rods 42 are in contact with the target tissue area, the electrodes 410 on the some of the bearing rods 42 with opposite polarities are conducted to transfer ablation energy to the target tissue area. In some embodiments, the electrodes 410 with opposite polarities are all located on the same bearing rod 42. In other embodiments, the electrodes 410 with opposite polarities are located on multiple spaced bearing rods 42.

Specifically, please referring to Fig. 7, the multiple first electrodes 411 forming the first ring 412 are selected as positive pole, and the multiple second electrodes 413 forming the second ring 414 are selected as negative pole. Of course, in other embodiments, the multiple first electrodes 411 forming the first ring 412 can also be selected as negative pole, and the multiple second electrodes 413 forming the second ring 414 can be selected as positive pole. By detecting the impedance of each first electrode 411 and each second electrode 413, the attachment situation is determined. When only some of the bearing rods 42 are in contact with the target tissue area, the electrodes 410 with opposite polarities are selected from the some of the bearing rods 42 that are in contact with the target tissue area to participate in segmented discharge. For example, at least two electrodes 410 on a single bearing rod 42 with opposite polarities are conducted. Alternatively, at least two electrodes 410 on two bearing rods 42 with opposite polarities are conducted, for example, at least two electrodes 410 on two adjacent bearing rods 42 with opposite polarities can be conducted, so as to deliver ablation energy to a portion of the target tissue area. More specifically, when only some of the bearing rods 42 are in contact with the target tissue area, the first electrode 411 and the second electrode 413 on the same bearing rod 42 are conducted; or, the first electrode 411 and the second electrode 413 on two adjacent bearing rods 42 are conducted, or, the first electrode 411 and the second electrode 413 on two non-adjacent bearing rods 42 can be conducted, so as to deliver ablation energy to the target tissue area. Among them, the polarity of the first electrode 411 is opposite to that of the second electrode 413. Each first electrode 411 and a corresponding second electrode 413 form an ablation electrode couple. After the ablation is completed, all electrodes 410 on the support framework 201 participate in the mapping.

Please referring to Fig. 8, for ease of description, the two first bearing rods 421 located between two adjacent second bearing rods 422 are defined as the first sub bearing rod 4211 and the second sub bearing rod 4212, respectively . The first sub bearing rod 4211 is provided with two electrodes 410, and the second sub bearing rod 4212 is provided with one electrode 410. The electrodes 410 on the first sub bearing rod 4211 are selected as the positive pole, and the electrode 410 on the second sub bearing rod 4212 are selected as the negative pole. Of course, in other embodiments, the electrodes 410 on the first sub bearing rod 4211 can also be selected as the negative pole, and the electrode 410 on the second sub bearing rod 4212 can be selected as the positive pole. By detecting the impedance of each electrode 410, the attachment situation is determined. When only some of the bearing rods 42 are in contact with the target tissue area, the electrodes 410 with opposite polarities are selected from the some of the bearing rods 42 that are in contact with the target tissue area to participate in segmented discharge. For example, the electrodes 410 with opposite polarities on two adjacent bearing rods 42 are selected to transmit ablation energy to the target tissue area. Specifically, when only some of the bearing rods 42 are in contact with the target tissue area, the electrodes 410 on the first sub bearing rod 4211 and the electrode 410 on the second sub bearing rod 4212 are conducted. Optionally, the electrodes 410 on the first sub bearing rod 4211 and the electrode 410 on the second sub bearing rod 4212 adjacent to the first sub bearing rod 4211 are conducted. Among them, the polarity of electrodes 410 on the first sub bearing rod 4211 is opposite to that of electrode 410 on the second sub bearing rod 4212. After the ablation is completed, all electrodes 410 on the support framework 201 participate in the mapping.

### Embodiment 2

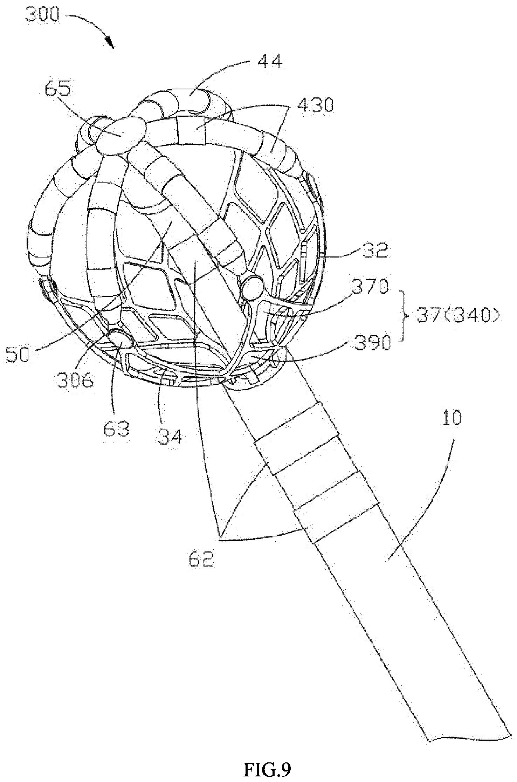

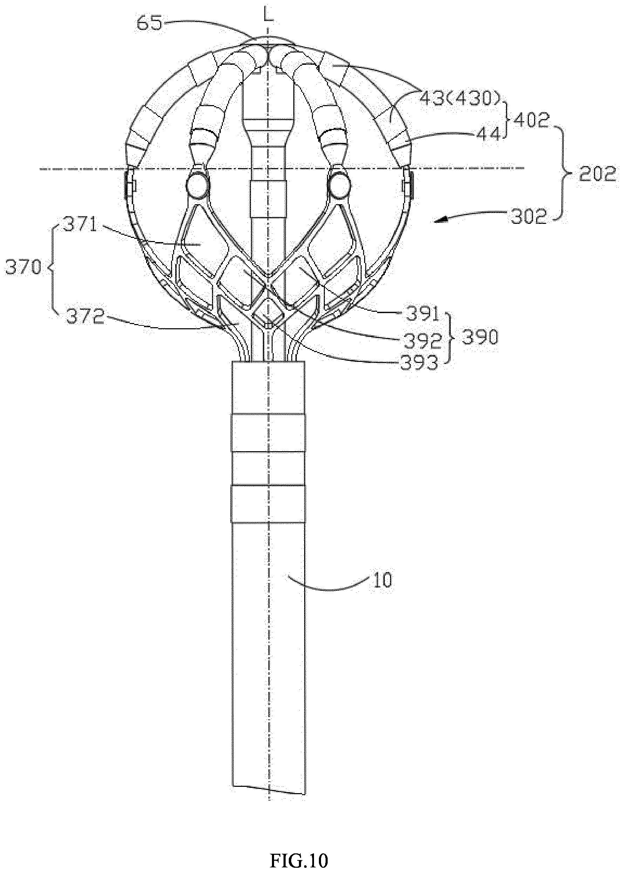

Please referring to Fig. 1, Fig. 9, and Fig. 10, Fig. 9 is a schematic view of the ablation catheter 300 provided by the second embodiment of the present application from a first perspective, and Fig. 10 is a schematic view of the ablation catheter 300 of Fig. 9 from a second perspective. The difference between the ablation catheter 300 provided in the second embodiment of the present application and the ablation catheter 100 provided in the first embodiment is in the structure of the support framework 202 and the arrangement of the electrodes 430 on the bearing rod 44. In addition, the ablation catheter 300 provided by the second embodiment further includes a traction member 50, a microelectrode 63, and a head electrode 65, wherein a reference electrode 62 is further provided on the traction member 50. In some embodiments, the reference electrode 62 may also be only provided on the traction member 50. It should be noted that the remaining structure of the ablation catheter 300 provided in the second embodiment is the same as that of the first embodiment, and will not be repeated here.

The connecting frame 302 of the support framework 202 is a mesh structure 340 formed by connecting multiple connecting rods 34 end-to-end. The connecting frame 302 of the support framework 202 includes multiple mesh units 37 set around the central axis L of the catheter body 10. Each mesh unit 37 includes at least one mesh. In this embodiment, each mesh unit 37 includes two main meshes 370, namely a first main mesh 371 and a second main mesh 372, respectively. The first main mesh 371 and the second main mesh 372 share one mesh node. Two adjacent second main meshes 372 share one side with each other. The second main mesh 372 is an open polygon, and its proximal end is connected to the distal end of the catheter body 10. There is a connecting mesh 390 set between two adjacent mesh units 37, which shares one side with each of two first main meshes 371 and two second main meshes 372 of the adjacent two mesh units 37, respectively. The proximal end of the first main mesh 371 is connected to the proximal end of the bearing frame 402. The proximal end of the second main mesh 372 is connected to the distal end of the catheter body 10 by means of but not limited to one or a combination of welding, clamping, bonding, thermal fusion, threaded connection, sealing connection, etc. The specific connection manner should be selected according to the usage requirements or functional requirements. In this embodiment, the support framework 202 is also made by cutting and heat setting a nickel titanium alloy pipe, that is, the proximal end of the first main mesh 371 is integrated with the proximal end of the bearing frame 402. In other embodiments, the proximal end of the first main mesh 371 and the proximal end of the bearing frame 402 may also be connected by one or a combination of welding, clamping, bonding, thermal fusion, threaded connection, sealing connection, etc.

In one embodiment, the connecting mesh 390 includes a first sub-mesh 391, a second sub-mesh 392, and a third sub-mesh 393. The first sub-mesh 391, the second sub-mesh 392, and the third sub-mesh 393 share one mesh node. The third sub-mesh 393 shares one side with the first sub-mesh 391 and the second sub-mesh 392, respectively. The first sub-mesh 391 shares one side with one of the first main meshes 371 of the two adjacent mesh units 37, and the second sub-mesh 392 shares one side with the other first main mesh 371 of the two adjacent two mesh units 37. The first sub-mesh 391 and the third sub-mesh 393 together shares one side with one of the second main meshes 372 of the two adjacent mesh units 37, and the second sub-mesh 392 and the third sub-mesh 393 together shares one side with the other second main mesh 372 of the two adjacent mesh units 37.

Among them, the profiles of the main mesh 370 and the connecting mesh 390 are both polygons, such as diamonds, trapezoids, pentagons, hexagons, or other polygons. In this embodiment, the first main mesh 371 is diamond-shaped, the second main mesh 372 has an open diamond shape, and the first sub-mesh 391, the second sub-mesh 392, and the third sub-mesh 393 are all diamond-shaped. The connecting frame 302 of the support framework 202 includes thirty meshes, all of which are diamond-shaped. It can be understood that the shape and number of the meshes are not limited in this application. The shape of the meshes can also be other polygons, and, the number of the meshes can be set according to actual needs.

The bearing frame 402 of the support framework 202 provided by the second embodiment is similar to the bearing frame 402 of the support framework 202 provided by the first embodiment in structure, except that in the second embodiment, there are six bearing rods 44 arranged in the circumferential direction around the central axis L of the conduit body 10. On any bearing rod 44, there is at least one electrode 430, that is, at least one electrode 430 spaced along the axial direction is provided on each bearing rod 44. On the multiple bearing rods 44, the electrodes 430 correspond one-to-one along the axial direction of the catheter body 10 and are arranged at circumferential intervals around the central axis L of the support framework 202. In this embodiment, each bearing rod 44 is provided with two electrodes 430. The two electrodes 430 both are annular electrodes. Each electrode 430 can be used as an ablation electrode or as a mapping electrode.

In this embodiment, the multiple electrodes 430 include multiple first electrodes 431 and multiple second electrodes 433. Each bearing rod 44 is provided with the first electrode 431 and the second electrode 433. The multiple first electrodes 431 form a first ring 432 in the circumferential direction of the bearing frame 402, and the multiple second electrodes 433 form a second ring 434 in the circumferential direction of the bearing frame 402. The first ring 432 is relatively close to the proximal end of the bearing frame 402, and the second ring 434 is relatively close to the distal end of the bearing frame 402. The plane on which the first ring 432 is located and the plane on which the second ring 414 is located are both perpendicular to the central axis L of the catheter body 10. Among them, the plane on which the first ring 432 is located and the plane o which the second ring 414 is located are parallel.

In this embodiment, the ablation catheter 300 further includes a traction member 50. The traction member 50 is movably arranged in the catheter body 10 and extends out from the distal end of the catheter body 10. The distal ends of the multiple bearing rods 44 are gathered and connected to the distal end of the traction member 50. Due to the distal end of the support framework 202 being restrained at the distal end of the traction member 50, and the traction member 50 extends along the catheter body 10 to connect the handle, it is possible to control the contraction and expansion of the support framework 202 through the traction member 50.

Optionally, the traction member 50 is coaxially inserted into the catheter body 10, with the distal end of the traction member 50 extending out of the distal end of the catheter body 10 and connecting to the distal end of each bearing rod 44. The proximal end of the traction member 50 is connected to the handle, which pulls the traction member 50 and drives it to move axially, thereby enabling the traction member 50 to move axially relative to the catheter body 10 and drive the support framework 202 to expand or contract radially.

It can be understood that the traction member 50 can move relative to the catheter body 10 along the central axis L of the traction member 50. During the process of the traction member 50 moving from the distal end to the proximal end of the catheter body 10, the axial size of the support framework 202 decreases and the radial size of the support framework 202increases. During the process of the traction member 50 moving from the proximal end to the distal end of the catheter body 10, the axial size of the support framework 202 increases and the radial size of the support framework 202 decreases. Specifically, when the traction member 50 moves relative to the catheter body 10 from the distal end to the proximal end, the distal ends of the bearing rods 44 move along with the traction member 50 towards the proximal end of the catheter body 10, the middle portions of the bearing rods 44 will gradually expand radially and outwardly. As a result, the outer diameter of the support framework 202 will increase, that is, the support framework 202 expands outward. On the contrary, when the traction member 50 moves relative to the catheter body 10 from the proximal end to the distal end, the distal ends of the bearing rods 44 move along with the traction member 50 towards the distal end, causing the bearing rods 44 to be straightened. The middle portions of the bearing rods 44 gradually approach the central axis L of the catheter body 10 in the radial direction, thereby reducing the outer diameter of the support framework 202. Thus, the outer diameter of the support framework 202 can be flexibly adjusted through the traction member 50. On one hand, this allows the support framework 202 to adapt to blood vessels of different diameters (such as pulmonary veins) or other body tissues, and can ablate the target ablation area under any appropriate outer diameter size, rather than being limited to the situation where the support framework 202 must undergo ablation under the maximum axial compression degree (i.e., the maximum radial expansion degree). This improves the adaptability to the anatomical morphology of different target ablation areas, facilitates the operation of the ablation device, and achieves better ablation effects. On the other hand, by adjusting the outer diameter of the support framework 202 through the traction member 50, the outer diameter can be reduced, which facilitates the retraction and release operations of the support framework 202, making the retraction and release operations faster and more convenient. At the same time, it avoids damage to the bearing rods 44 and the electrodes 430 during the retraction process.

In one embodiment, when the bendable section 11 undergoes bending, in order to prevent changes in the radial size of the support framework 204 and the linkage between the bending function and the diameter adjustment function, the bendable section 11 can be configured as a hypotube. It should be noted that hypotube is a long metal tube with micro engineering characteristics throughout the entire pipeline of the hypotube. A plurality of cutting grooves spaced from each other in the axial direction is defined in the bendable section 11 of the catheter body 10 to form the hypotube. For example, in some embodiments, the cutting grooves may be configured as spiral grooves. In other embodiments, the multiple cutting grooves are configured as annular grooves and staggered along the circumferential direction of the catheter body 10.

In some embodiments, the traction member 50 may also be omitted, that is, the ablation catheter 300 may not be configured with the traction member 50, and the support framework 202 can expand radially in a self-expanding manner. At this time, the diameter of the support framework 202 cannot be adjusted and can be used for ablation target areas with fixed size. In addition, for the structure with the traction member 50 in this embodiment, the expansion degree of the support framework 202 can be adjusted by manipulating the traction member 50 to better fit the human tissue.

The traction member 50 may be made of steel cable, flexible polyimide pipe, fluorinated polyethylene pipe, stainless steel pipe, or other polymer materials. When the traction member 50 is configured as a tubular structure, it has a certain structural strength, which can buffer the external force received by the bearing rods 44 and effectively ensure the stability of the positions of the bearing rods 44.

In this embodiment, a reference electrode 62 is also provided on the pipe wall of the traction member 50. The connection manner between the reference electrode 62 and the traction member 50 includes but is not limited to welding, crimping, bonding, integrated molding, and etc. There is an opening for extending of the wire defined in the traction member 50 at a position corresponding to the reference electrode 62. The wire with an insulating layer is welded to the inner wall of reference electrode 62. One end of the wire is welded to the connector of the handle, and the other end extends along the lumen of the catheter body 10 and the inner cavity of the traction member 50,through the opening to connect the inner wall of the reference electrode 62. The number of reference electrodes 62 includes three, of which two reference electrodes 62 are set on the catheter body 10, and the remaining one reference electrode 62 is set on the traction member 50. It should be noted that the number of the reference electrodes 62 is only for illustration purposes and is not specifically limited in this application. For example, the number of reference electrodes 62 can also be set according to actual needs.

In some embodiments, the ablation catheter 300 further includes a microelectrode 63 for detecting electrocardiogram signals. The microelectrode 63 is set at the farthest end of the connecting frame 302 and is insulated from the conducting portion 32 and the electrode unit 43. The connecting frame 302 is provided with a mounting hole 306 for installing the microelectrode 63, so that the microelectrode 63 can be embedded in the main body of the connecting frame 302. That is, when the support framework 202 is in a contracted state, the radial size of the connecting frame 302 at the microelectrode 63 is approximately equal to the radial size of the portion of the connecting frame 302 outside the microelectrode 63, which facilitates the recovery of the support framework 202 into or extending from the sheath catheter 21. The mounting hole 306 may be a through hole that penetrates the connecting frame 302, or a blind hole that does not penetrate the connecting frame 302. The microelectrode 63 and the connecting frame 302 can be bonded together by glue, thereby achieving electrical isolation from each other through the glue. For example, the microelectrode 63 is fixed at the farthest end of the connecting frame 302 using medical insulating glue. Alternatively, a non-metallic insulator can be added between the microelectrode 63 and the farthest end of the connecting frame 302 to achieve mutual isolation. The material of the microelectrode 63 includes metal, used for detecting electrocardiogram signals. The inner side of the microelectrode 63 is connected to a wire. The wire of the microelectrode 63 and the wire of the electrode 430 on the bearing rod 44 can share a common wire path. Of course, it can be understood that the wire of the microelectrode 63 can also be set in a wire path different from that of the electrode 430.

Specifically, in this embodiment, the ablation catheter 300 includes six microelectrodes 63. It can be understood that the number of the microelectrodes 63 in this application is not specifically limited. For example, the number of the microelectrodes 63 can also be set according to actual needs. For example, the number of the microelectrodes 63 can be set according to the number of the first main meshes 371, being less than or equal to the number of the first main meshes 371.

In some embodiments, the ablation catheter 300 further includes a head electrode 65 set on the farthest end of the traction member 50. The head electrode 65 is used to cooperate with the conducting portion 32 and the multiple electrodes 430 to release ablation energy, as well as for electrocardiogram signal mapping. There is a slot defined in the distal end face of the traction member 50, and the head electrode 65 is inserted into the slot, making it convenient for later replacement or disassembly of the head electrode 65 and easy to use. Optionally, the distal end of the bearing frame 402 and the distal end of the head electrode 65 are arc-shaped and smoothly connected. Specifically, the distal end face of the support framework 202 is arc-shaped, and the distal end face of the head electrode 65 is arc-shaped and tangent to the arc-shaped surface of the support framework 202. When the head electrode 65 is connected to the energy generator to transfer ablation energy to the target tissue area, the head electrode 65 is in the same plane as the arc-shaped surface at the distal end of the support framework 202, which enables the head electrode 65 to stably attach to the lesion tissue and perform ablation at any angle of the lesion tissue, thereby achieving fast, efficient, and high-quality ablation results. Optionally, the distal ends of the multiple bearing rods 44 of the bearing frame 402 can be combined together to form an arc-shaped structure, or a distal umbrella structure or other forms with an arc or circular structure, to avoid instrument damage to atrial tissue caused by the protruding tip of the traction member 50 and better conform to the ablation area of the heart. The head electrode 65 is connected to an insulated wire, one end of which is welded to the connector on the handle and the other end extends through the lumen of the catheter body 10 and the inner cavity of the traction member 50 to connect to the head electrode 65.

In this embodiment, the ablation catheter 300 can transfer ablation energy to the target tissue area by cooperating of the multiple electrodes 430 on the bearing frame 402, the head electrode 65, and the conducting portion 32 of the connecting frame 302. Specifically, the multiple electrodes 430 and the head electrode 65 are connected to the positive pole of the energy generator, and the conducting portion 32 of the connecting frame 302 is connected to the negative pole of the energy generator. Alternatively, the multiple electrodes 430 and the head electrode 65 can be connected to the negative pole of the energy generator, and the conducting portion 32 of the connecting frame 302 can be connected to the negative pole of the energy generator.

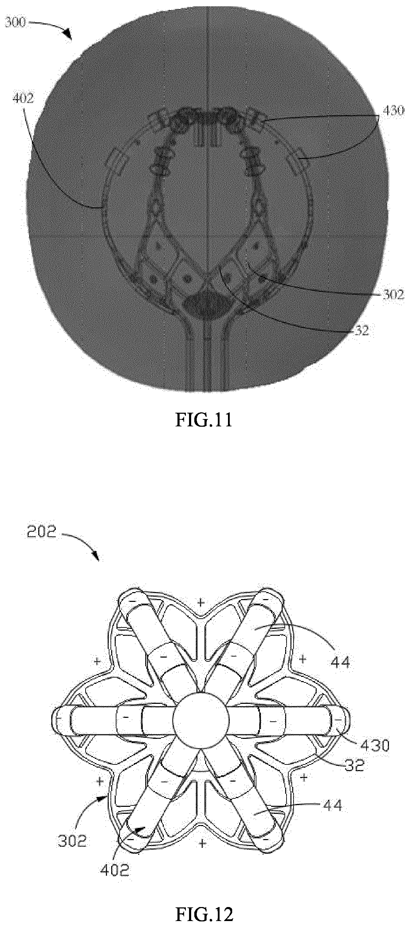

Please referring to Fig. 9 and Fig. 11, Fig. 11 is a simulation diagram of an electric field distribution of the ablation catheter 300 of Fig. 9. The conducting portion 32 of the connecting frame 302 is the positive pole, and the electrode 430 of the bearing frame 402 is the negative pole. The positive and negative poles of the ablation catheter 300 form a pulsed electric field as shown in Fig. 11 to transmit ablation energy to the target tissue area. When the electric field strength is 500V/cm, the maximum coverage width of the pulsed electric field between the conducting portion 32 of the connecting frame 302 and the electrodes 430 of the bearing frame 402 is about 6.5mm, which is greater than 6mm. The ablation depth exceeds 6mm, thus the mitral valve isthmus can be ablated through the wall to achieve therapeutic effect.

It can be understood that the ablation method of the ablation catheter 300 can be focal ablation discharge or other methods, as described in detail in the first embodiment. After the ablation is completed, all of the electrodes 430 and the head electrode 65 are used for potential mapping. Compared to the typical six or nine mapping electrodes, in this embodiment, all of the electrodes 430 and the head electrode 65 are used as mapping electrodes. The number of mapping electrodes can be greatly increased to thirteen, which can greatly improve the collection range and efficiency of electrophysiological signals, thereby making mapping more accurate. When the bearing frame 402 of the support framework 202 is attached to the myocardium, electrocardiogram conduction signals in multiple directions can be collected, making the collected potential signals more accurate. Meanwhile, utilizing all of the electrodes 430 and the head electrode 65 of the ablation catheter 300 in this embodiment enables efficient and rapid 3D modeling. The specific content of 3D mapping and modeling can refer to the first embodiment and will not be repeated here.

Please referring to Fig. 1 and Fig. 12, Fig. 12 is a schematic view of a first discharge mode for segmented discharge of the ablation catheter 300 of Fig. 9. In this embodiment, when only some of the bearing rods 44 are in contact with a portion of the target tissue area, the conducting portion 32 and at least one electrode 430 on the some of the bearing rods 44 are conducted, so as to transfer ablation energy to the portion of the target tissue area, wherein the at least one electrode 430 has a polarity opposite to that of the conducting portion 32.

Please referring to Fig. 12, the conducting portion 32 of the connecting frame 302 is selected as the positive pole, and the electrode 430 of the bearing frame 402 is selected as the negative pole. Alternatively, the conducting portion 32 can be selected as the negative pole and the electrode 430 of the bearing frame 402 can be selected as the positive pole. By detecting the impedance of each electrode 430, the attachment situation can be determined. When only some of the bearing rods 44 are in contact with the target tissue area, at least one electrode 430 from the some of the bearing rods 44 that are in contact with the target tissue area and the conducting portion 32 can be selected to participate in segmented discharge. For example, at least one electrode 430 on a single bearing rod 44 of the some of the bearing rods 44 that are in contact with the target tissue area and the conducting portion 32 can be selected to participate in segmented discharge. Alternatively, the electrodes 430 on at least two of the some of the supporting rods 44 that are in contact with the target tissue area and the conducting portion 32 can be selected to participate in segmented discharge. Optionally, the conducting portion 32 and the electrodes 430 on two adjacent supporting rods 44 can be selected to participate in segmented discharge. Among them, the polarity of the conducting portion 32 is opposite to that of the selected electrode 430. After the ablation is completed, all of the electrodes 430 on the support framework 202 and all microelectrodes 63 participate in the mapping.

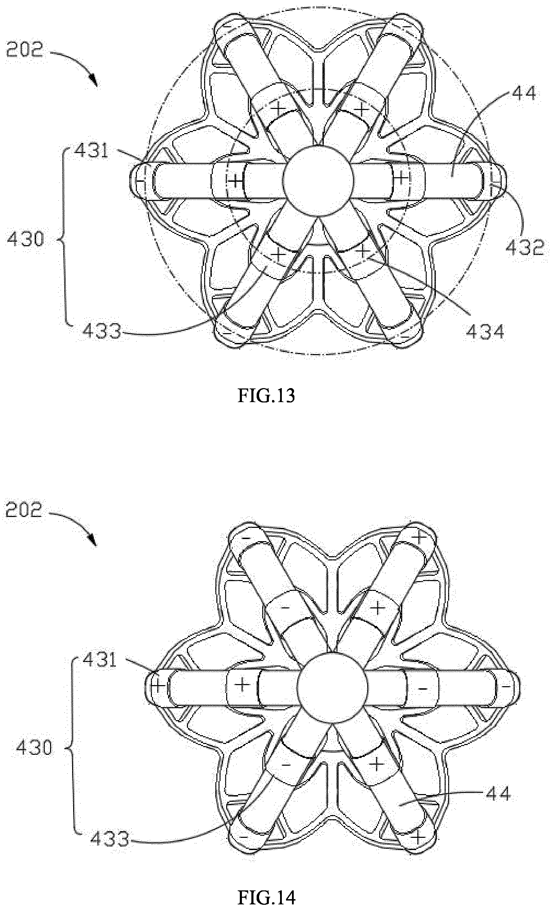

Please referring to Fig. 1, Fig. 13, and Fig. 14, Fig. 13 is a schematic view of a second discharge mode for segmented discharge of the ablation catheter 300 of Fig. 9, and Fig. 14 is a schematic view of a third discharge mode for segmented discharge of the ablation catheter 300 of Fig. 9. When only some of the bearing rods 44 are in contact with the target tissue area, the electrodes 430 on the some of the bearing rods 44 with opposite polarities are selected to transfer ablation energy to the target tissue area. In some embodiments, the electrodes 430 with opposite polarities are all located on the same bearing rod 44. In other embodiments, the electrodes 430 with opposite polarities are located on multiple spaced bearing rods 44.

Please referring to Figs. 9 and 13, the multiple first electrodes 431 forming the first ring 432 are selected as positive pole, and the multiple second electrodes 433 forming the second ring 434 are selected as negative pole. Alternatively, the multiple first electrodes 431 that form the first ring 432 can be selected as negative pole, and the multiple second electrodes 433 that form the second ring 434 can be selected as positive pole. By detecting the impedance of each first electrode 431 and each second electrode 433, the attachment situation can be determined. When only some of the bearing rods 44 are in contact with the target tissue area, the electrodes 430 on the some of the bearing rods 44 that are in contact with the target tissue area with opposite polarities can be selected to participate in segmented discharge. For example, the first electrodes 431 and the second electrodes 433 on a single bearing rod 44 are conducted. Alternatively, the first electrodes 431 and the second electrodes 433 on multiple bearing rods 44 can be conducted. Optionally, the first electrode 431 and the second electrode 433 on two adjacent bearing rods 44 can be conducted to transfer ablation energy to the target tissue area. Among them, the polarity of the first electrode 431 is opposite to that of the second electrode 433. Each first electrode 431 and a corresponding second electrode 433 form an ablation electrode couple. After the partition ablation of the target tissue area is completed, all of the electrodes 430 on the support framework 202 and all microelectrodes 63 participate in the mapping.

Please referring to Fig. 9 and Fig. 14, the electrodes 430 on two adjacent bearing rods 44 of the multiple bearing rods 44 with opposite polarities are selected. By detecting the impedance of each electrode 430, the attachment situation is determined. When only some the bearing rods 44 are in contact with the target tissue area, the electrodes 430 on the some of the bearing rods 44 that are in contact with the target tissue area with opposite polarities are selected to participate in segmented discharge. Specifically, electrodes 430 with opposite polarities on at least two of the some of the bearing rods 44 that are in contact with the target tissue area are conducted. For example, at least two electrodes 430 with different polarities on two adjacent bearing rods 44 of the some of the bearing rods 44 are conducted, so as to transfer ablation energy to the target tissue area. After the ablation is completed, all of the electrodes 430 on the support framework 202 and all microelectrodes 63 participate in the mapping.

It should be noted that the structural design of the connecting frame 302, traction member 50, reference electrode 62, microelectrode 63, and head electrode 65 of the ablation catheter 300 in the second embodiment is applicable to the ablation catheter 100 in the first embodiment, and will not be repeated here.

### Embodiment 3

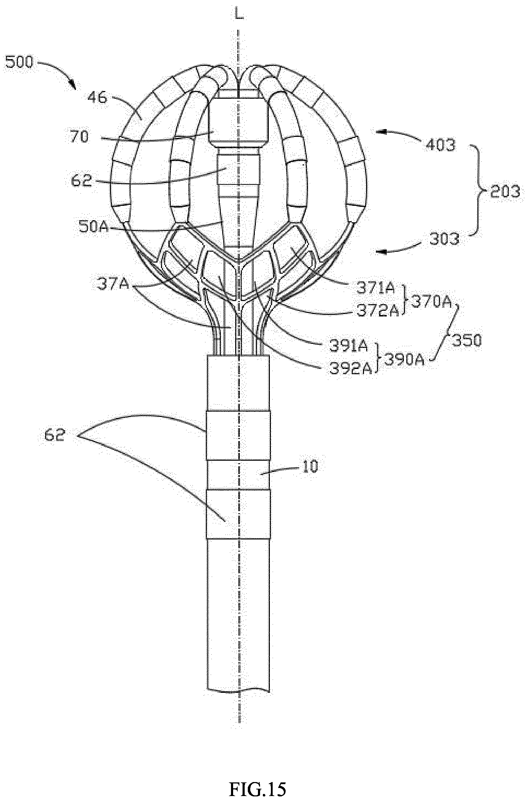

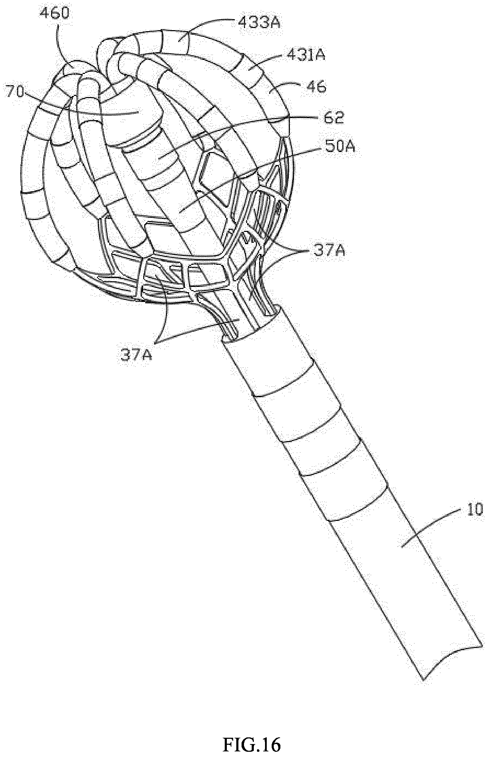

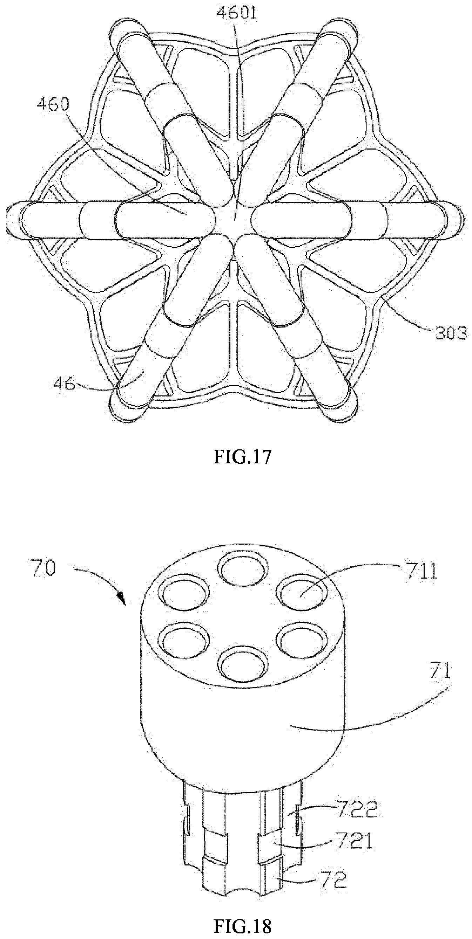

Please referring to Fig. 9 and Figs. 15-17, Fig. 15 is a schematic view of an ablation catheter provided in a third embodiment of the present application from a first perspective; Fig. 16 is a schematic view of the ablation catheter of Fig. 15 from a second perspective; and Fig. 17 is a schematic view of the ablation catheter of Fig. 15 from a third perspective. The difference between the ablation catheter 500 provided in the third embodiment of the present application and the ablation catheter 300 provided in the second embodiment is in that: the connection manner between the bearing frame 403 of the support framework 203 and the traction member 50A is different from that between the bearing frame 402 and the traction member 50 in the second embodiment, the mesh structure 350 of the connecting frame 303 of the support framework 203 is different from the mesh structure 340 in the second embodiment, the support framework 203 does not include microelectrodes and head electrodes, that is, microelectrodes are not provided on the conducting portion 32A, and head electrodes are not provided on the farthest end of the traction member 50A. It should be noted that the remaining structure of the ablation catheter 500 provided by the third embodiment is the same as that of the second embodiment, and will not be repeated here. For example, the ablation catheter 500 includes a reference electrode 62.

In the third embodiment, the mesh structure 350 includes multiple mesh units 37A and connecting meshes 390A connected between adjacent two mesh units 37A. The structure of the multiple mesh units 37A is the same as that of the multiple mesh units 37 in the second embodiment, and will not be repeated here. The difference is that the connecting mesh 390A includes a first sub-mesh 391A and a second sub-mesh 392A. The first sub-mesh 391A and the second sub-mesh 392A share one side with each other, and the first sub-mesh 391A shares one side with the first main mesh 371A and the second main mesh 372A in the same mesh unit 37A, respectively. The second main mesh 372A shares one side with the first main mesh 371A and the second main mesh 372A in the same mesh unit 37A, respectively. The free sides of the first main mesh 371A, the first sub-mesh 391A, the second sub-mesh 392A, and the second main mesh 372A, which are sequentially connected to each other, smoothly transition to form a roughly V-shaped structure, thereby avoiding instrument damage to atrial tissue caused by the mesh structure 350. The distal edge of the mesh structure 350 forms a petal-like structure. The first sub-mesh 391A and the second sub-mesh 392A are closed loop polygons.

The distal end of the bearing frame 403 is connected to the distal end of the traction member 50A, thereby achieving fast and convenient retraction and release operations of the ablation catheter 500 under the traction of the traction member 50A. The farthest end of the traction member 50A does not exceed the farthest end of the bearing frame 403, that is, the farthest end of the traction member 50A is located within the axial profile range of the bearing frame 403, which can avoid the formation of protruding tips at the distal end of the ablation catheter 500 and cause instrument damage to the atrial tissue, thus better conforming to the ablation area of the heart.

Optionally, in this embodiment, the distal end of the bearing frame 403 is provided with a reverse portion 460. The structural design of this reverse portion 460 can be used for ablation therapy in patients with myocardial hypertrophy. Specifically, the distal end of the bearing rod 46 is formed with the reverse portion 460. The reverse portion 460 bends from the farthest end of the bearing rod 46 towards the proximal end and extends gradually towards the central axis of the support framework 203. The proximal end of the reverse portion 460 is connected to the distal end of the traction member 50A. The reverse portion 460 is concaved along the central axis of the support framework 203 to form a bowl-haped concave area 4601, thereby reserving space for the target tissue area when subjected to the force of the support framework 203, avoiding displacement of the support framework 203, and achieving stable ablation of the ablation catheter 500 during operation.

It should be noted that the length of the reverse portion 460 can be set as needed, which can change the shape of the distal end of the support framework 203 to adapt to different cardiac ablation areas.

Optionally, the first electrode 431A on the bearing rod 46 is placed near the connecting frame 303, and the second electrode 433A is placed near the reverse portion 460, so that during ablation of the ablation catheter 500, the second electrode 433A can be closer to the target tissue area, which is beneficial for improving ablation quality and efficiency.

In some embodiments, the distal end of the bearing frame 403 may not be provided with a reverse portion 460, that is, the distal end of the bearing frame 403 and the distal end of the traction member 50 are arc-shaped and smoothly connected to each other. Specifically, the distal end face of the support framework 203 is arc-shaped, and the distal end face of the traction member 50 is arc-shaped and tangent to the arc-shaped surface of the support framework 203, thereby enabling the support framework 203 to stably attach to the lesion tissue and improving the ablation quality of the ablation catheter 500.

Please referring to Fig. 15 and Fig. 18, Fig. 18 is a schematic view of a connecting member of the ablation catheter 500 of Fig. 15. In this embodiment, the ablation catheter 500 further includes a connecting member 70 connected to the reverse portion 460 and the traction member 50A. One end of the connecting member 70 is fixedly connected to the reverse portion 460, and the other end is fixedly connected to the traction member 50A. Specifically, the bearing frame 403 includes multiple bearing rods 46. The connecting member 70 includes a first connecting portion 71 connected to the reverse portion 460 and a second connecting portion 72 connected to the first connecting portion 71 and the traction member 50A. The distal end of the first connecting portion 71 is provided with multiple positioning holes 711 extending along the axial direction, and the multiple bearing rods 46 are fixed in corresponding positioning holes 711, respectively, to achieve positioning and fixation of the bearing rods 46. Optionally, the multiple positioning holes 711 are uniformly arranged along the circumference of the first connecting portion 71 to avoid uneven spacing between adjacent bearing rods 46, which can lead to uneven electric fields between different regions of the spherical support framework 203. This can achieve more uniform ablation of the target tissue area by the ablation catheter 500 and improve the ablation effect.

The second connecting portion 72 is inserted into an interior of the traction member 50A. In this embodiment, the second connecting portion 72 is fixedly connected to the traction member 50A by adhesive bonding. In other embodiments, the second connecting portion 72 and the traction member 50A may also be fixed together by clamping, welding, etc., which is not limited in this application.

Specifically, the second connecting portion 72 is provided with a first groove 721 extending circumferentially to accommodate the adhesive, thereby enhancing the connection strength between the second connecting portion 72 and the traction member 50A, as well as ensuring the stability and reliability of the connection between the second connecting portion 72 and the traction member 50A. Optionally, the second connecting portion 72 is also provided with multiple second grooves 722 extending axially, which are spaced from each other and communicated with the first groove 721, so as to ensure that the connecting member 70 is connected to the traction member 50A in the circumferential and axial directions, prevent displacement of the connecting member 72 in the axial and circumferential directions, and enhance the stability of the connection. In some embodiments, the second connecting portion 72 may also have grooves configured in spiral, wavy, S-shaped, Z-shaped, and etc. along the axial and/or circumferential directions. The bottom of the first groove 721 and the groove wall of the second groove 722 are arc-shaped, increasing the contact area between the adhesive and the second connecting portion 72 and traction member 50A, further enhancing the connection stability between the connecting member 70 and the traction member 50A.

Optionally, the second connecting portion 72 is inserted into the interior of the traction member 50A, and the first connecting portion 71 is blocked by the traction member 50A. Specifically, the radial size of the first connecting portion 71 is larger than that of the second connecting portion 72, and the radial size of the second connecting portion 72 is approximately equal to or smaller than the radial size of an inner cavity of the traction member 50A. Therefore, when the second connecting portion 72 is inserted into the traction member 50A, the first connecting portion 71 is blocked by the traction member 50A, thereby improving the assembly efficiency of the connecting portion 70 with the traction member 50A and the bearing frame 403.

### Embodiment 4

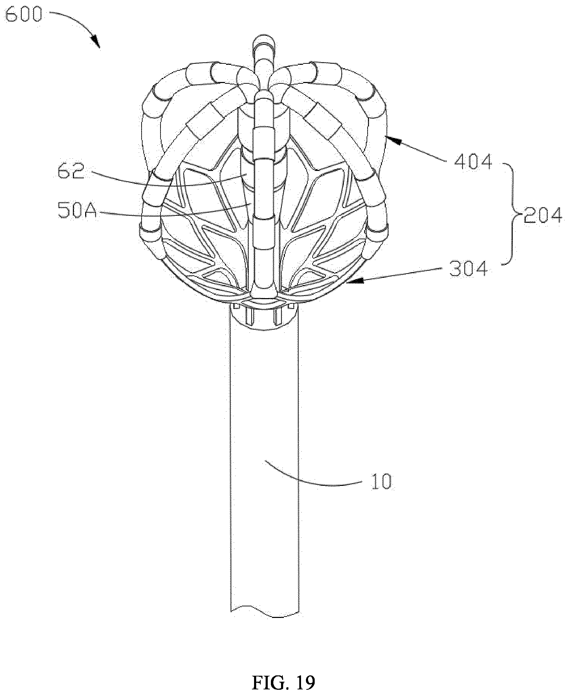

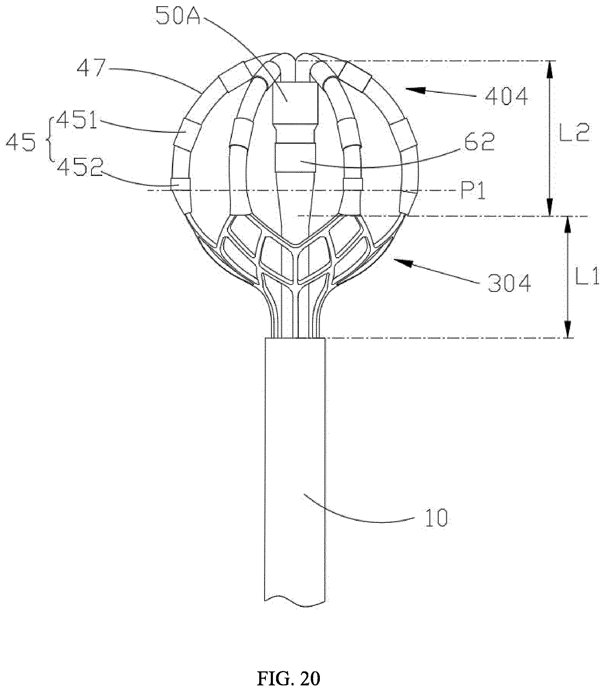

Please referring to Figs. 19-20, Fig. 19 is a schematic view of an ablation catheter 600 provided in a fourth embodiment of the present application from a first perspective, and Fig. 20 is a schematic view of the ablation catheter 600 of Fig. 19 from a second perspective. The support framework 204 includes a connecting frame 304 and a bearing frame 404 that are connected to each other in the axial direction. The proximal end of connecting frame 304 is connected to the distal end of catheter body 10, and the distal end of connecting frame 304 is connected to the proximal end of bearing frame 404. The difference between the ablation catheter 600 provided in the fourth embodiment of the present application and the ablation catheter 500 provided in the third embodiment is that: the axial length L1 of the connecting frame 304 is smaller than the axial length L2 of the bearing frame 404. The ablation catheter 600 further includes at least one mapping electrode 452 arranged on the bearing frame 404. It should be noted that the remaining structure of the ablation catheter 600 provided by the fourth embodiment is the same as that of the third embodiment, and will not be repeated here. For example, the ablation catheter 600 includes a reference electrode 62 and a traction member 50A.

In this embodiment, the electrode unit 45 set on the bearing frame 404 includes multiple ablation electrodes 451 to achieve ablation of the target tissue area. The bearing frame 404 includes multiple bearing rods 47. The axial length of the multiple bearing rods 47 is the same. Multiple ablation electrodes 451 are provided on each bearing rod 47 and spaced from each other along the axial direction. The support framework 204 has an equatorial position P1 located between the proximal end and the distal end of the support framework 204. The ablation electrode 451 is located between the equatorial position P1 and the distal end of the bearing frame 404. It should be noted that the equatorial position P1 of the support framework 204 refers to a position perpendicular to the central axis of the support framework 204 and equidistant from two axial ends of the support framework 204. By way of example, in this embodiment, the ablation electrode 451 is located between the equatorial position P1 of the support framework 204 and the distal end of the bearing frame 404, which is beneficial for improving the ablation depth of the ablation catheter 600. Among the multiple bearing rods 47, the ablation electrodes 451 correspond one by one along the axial direction and are arranged at circumferential intervals around the axis of the catheter body 10. In this embodiment, each bearing rod 47 is provided with two ablation electrodes 451. The ablation electrode 451 is configured as an annular electrode. Among the multiple bearing rods 47, the ablation electrodes 451 correspond one-to-one along the axial direction. All of the ablation electrodes 451 mentioned above can serve as mapping electrodes to participate in the mapping of electrocardiogram signals.

When the support framework 204 is attached to the myocardial tissue, and the ablation electrode 451 on the bearing rod 47 is placed between the equatorial position P1 of the support framework 204 and the distal end of the support framework 404, the ablation electrode 451 may be in a suspended state and cannot accurately and reliably map the electrocardiogram signals of the myocardial tissue before and after ablation. In some embodiments of the present application, the electrode unit 45 provided on the bearing frame 404 further includes at least one mapping electrode 452. The at least one mapping electrode 452 is located on the equatorial position P1 of the support framework 204, or between the equatorial position P1 and the proximal end of the bearing frame 404. The mapping electrode 452 is used to detect electrophysiological signals in the target tissue area. The mapping electrode 452 located on the equatorial position P1 of the support framework 204 means that the equatorial position P1 of the support framework 204 is located between the distal end and the proximal end of the mapping electrode 452, or the equatorial position P1 of the support framework 204 coincides with the distal end of the mapping electrode 452, or the equatorial position P1 of the support framework 204 coincides with the proximal end of the mapping electrode 452. The mapping electrode 452 located between the equatorial position P1 of the support framework 204 and the proximal end of the bearing frame 404 may indicate that the distal end of the mapping electrode 452 is spaced from the equatorial position P1 of the support framework 204. In some embodiments, all mapping electrodes 452 are positioned at the equatorial position P1 of the support framework 204, or between the equatorial position P1 and the proximal end of the bearing frame 404, ensuring accurate mapping of myocardial electrocardiogram signals even when the support framework 204 is laterally attached to myocardial tissue.

Multiple mapping electrodes 452 are arranged on different bearing rods 47. For example, in this embodiment, the electrode unit 45 includes three mapping electrodes 452 for detecting electrocardiogram signals. The three mapping electrodes 452 are respectively set on three non-adjacent bearing rods 47. The purpose of this setting is that: on one hand, compared to the scheme of setting mapping electrodes 452 on each bearing rod 47, setting mapping electrodes 452 on non-adjacent bearing rods 47 will reduce the number of mapping electrodes 452, which means that the number of wires will be less, which is more conducive to assembly in the process; on the other hand, mapping electrodes 452 are installed on non-adjacent bearing rods 47, and the number of mapping electrodes 452 is already sufficient to meet the mapping requirements. The position of each mapping electrode 452 is located at the proximal end of the bearing rod 47, close to the equatorial position P1 of the support framework 204, or between the equatorial position P1 and the proximal end of the bearing rod 47. The mapping electrode 452 is an annular electrode. In this embodiment, the shape of the mapping electrode 452 is not limited, and the mapping electrode 452 can also be a sheet electrode, a point electrode, or a spherical electrode. The material of the mapping electrode 452 can be medical metals such as platinum, iridium, gold, silver, etc., that can be used for interventional therapy. It can be understood that the length of the mapping electrode 452 cannot be too large. If the length of the mapping electrode 452 is too large, it will cause the potential signal collected by the mapping electrode 452 to become a composite signal, which is not conducive to the surgeon's judgment. In this embodiment, the length of the mapping electrode 452 cannot be too large, and the length of the mapping electrode 452 may be less than or equal to the length of the ablation electrode 451. The inner side of the mapping electrode 452 is connected to a wire, and the wire of the mapping electrode 452 and the wire of the ablation electrode 451 can share a common wire path. Of course, it can be understood that the wire of the mapping electrode 452 can also be set in a wire path different from that of the ablation electrode 451.

The connecting frame 304 is connected to the connector on the handle through a connecting wire (not shown in the figures). Specifically, one end of the connecting wire is connected to the connecting frame 304, and the other end of the connecting wire is connected to the connector on the handle through the lumen of the catheter body 10. It can be understood that due to the bendable function of the catheter body 10, the connecting wires located within the bendable section 11 of the catheter body 10 are prone to breakage. In some embodiments, the connecting wires located within the bendable section 11 of the catheter body 10 are configured in a spiral structure, which facilitates the bending operation of the catheter body 10, avoids the occurrence of tension and breakage of the connecting wires located within the bendable section 11 of the catheter body 10 due to insufficient margin, and also avoids the phenomenon of bending back after the bendable section 11 is adjusted. The spiral structure can alleviate the influence of extrusion force on the connecting wires, thereby avoiding the occurrence of wire breakage.

During pulse ablation, a pulsed electric field is formed between the positive and negative poles and acts on the target tissue area. In this embodiment, the connecting frame 304 which is conductive and configured in dense mesh form cooperates with the ablation electrode 451 to achieve ablation. Specifically, one of the connecting frame 304 and the ablation electrode 451 serves as the positive pole, and the other of the connecting frame 304 and the ablation electrode 451 serves as the negative pole. The connecting frame 304 is located in the lower hemisphere of the support framework 204, and the bearing frame 404 is located in the upper hemisphere of the support framework 204. The ablation electrode 451 is set between the equatorial position P1 of the support framework 204 and the distal end of the bearing frame 404. On one hand, the positive and negative pole positions are relatively dispersed, which can achieve a larger electric field coverage range between the positive and negative poles, thereby improving the depth of ablation damage; on the other hand, compared to the electrode area of the ablation electrode 451 on the bearing frame 404, the electrode area of the connecting frame 304 configured in dense mesh form is larger, so the current density of the connecting frame 304 is smaller, and the current density of the ablation electrode 451 on the bearing frame 404 is larger. Therefore, energy will be more concentrated in the bearing frame 404, which mainly attaches to the target tissue area for ablation. Therefore, in case of more concentrated energy, attaching the bearing frame 404 to the target tissue area for ablation makes the ablation depth deeper. In addition, the ablation electrode 451 can be used not only for ablation, but also for participating in mapping. Considering that the ablation electrode 451 may be in a suspended state when the support framework 204 is attached to the myocardial tissue, resulting in unsatisfactory mapping results, a mapping electrode 452 is installed near the equatorial position P1 of the spherical support framework 204 or between the equatorial position P1 and the proximal end of the bearing frame 404, so as to accurately map the electrocardiogram signal of the myocardium when the support framework 204 is attached to the myocardial tissue.

The ablation catheter 600 provided in this embodiment of the application is designed based on the connecting frame 304, the ablation electrode 451, and the mapping electrode 452, so that the ablation catheter 600 has both mapping and ablation functions. In the case of integrating mapping and ablation functions, the connecting frame 304 configured in dense mesh form is set in the lower hemisphere of the support framework 204, the ablation electrode 451 is set in the upper hemisphere of the support framework 204 and between the equatorial position P1 of the support framework 204 and the distal end of the support framework 404, and the mapping electrode 452 is set in the equatorial position P1 or between the equatorial position P1 and the proximal end of the support framework 404, so that the ablation catheter 600 can not only ensure ablation effect and improve ablation depth, but also improve mapping efficiency and effect, and ensure mapping accuracy.

In addition, it should be noted that the ablation catheter 600 of this embodiment can also perform partition ablation, achieving precise partition ablation. Through partition local ablation, it is possible to avoid damaging unexpected ablated tissues. The ablation energy can be targeted to the tissue that needs to be ablated, increasing the utilization rate of ablation energy, reducing the dissipation of ablation energy in the blood, and reducing unnecessary bubbles generated by electrolyzing blood.

### Embodiment 5

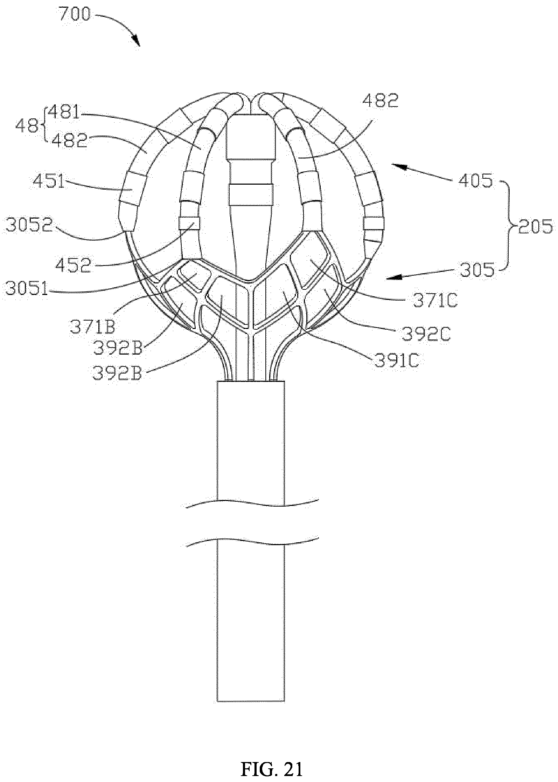

Please referring to Fig. 19 and Fig. 21, Fig. 21 is a schematic view of an ablation catheter 700 provided in a fifth embodiment of the present application. The support framework 205 includes a connecting frame 305 and a bearing frame 405 that are connected to each other in the axial direction. The proximal end of connecting frame 305 is connected to the distal end of catheter body 10, and the distal end of connecting frame 305 is connected to the proximal end of bearing frame 405. The bearing frame 405 includes multiple bearing rods 48. The difference between the ablation catheter 700 provided in the fifth embodiment of the present application and the ablation catheter 600 provided in the fourth embodiment is that: the multiple bearing rods 48 can be configured with different lengths. It should be noted that the remaining structure of the ablation catheter 700 provided by the fifth embodiment is the same as that of the fourth embodiment, and will not be repeated here.

It should be noted that due to the ablation electrode 451 being placed between the equatorial position P1 of the support framework 204 and the distal end of the support framework 405, and the mapping electrode 452 being placed on the equatorial position P1 of the support framework 204 or between the equatorial position P1 and the proximal end of the support framework 405, the axial length L1 of the connecting frame 304 is smaller than the axial length L2 of the support framework 404. The ablation depth is generally linearly related to the area ratio K between the surface area S_{cf} of the connecting frame 304 and the sum of the surface areas Sₐₑ of the multiple ablation electrodes 451. That is, if a larger ablation depth is desired, the area ratio K can be increased, wherein K = S_{cf} / Sₐₑ. The decrease in axial length of the connecting frame 304 means a decrease in S, that is, a decrease in K, resulting in a decrease in ablation depth.

In order to avoid any impact on the ablation depth caused by the setting of the mapping electrode 452, the lengths of the multiple bearing rods 48 can be set to be unequal. For example, in this embodiment, the multiple bearing rods 48 include multiple first rods 481 and multiple second rods 482. The multiple first rods 481 and multiple second rods 482 are alternately arranged around the central axis of the support framework 205, facilitating the retraction or release of the support framework 205. There are multiple mapping electrodes 452. Each first rod 481 is provided with at least one mapping electrode 452. For example, in this embodiment, the multiple bearing rods 48 include three first rods 481 and three second rods 482, each of which is provided with a mapping electrode 452. In some embodiments, the number of the first rods 481 may be less than the number of the second rods 482. The number of the first rods 481 may be one, two, more than three, and etc. Each first rod 481 may be provided with multiple mapping electrodes 452.

In some embodiments, an end of the connecting frame 305 facing the bearing frame 405 includes a first connecting end 3051 and a second connecting end 3052. The first connecting end 3051 is closer to the proximal end of the connecting frame 305 than the second connecting end 3052. The length of the first rod 481 is greater than that of the second rod 482. The proximal end of the first rod 481 is connected to the first connecting end 3051, and the proximal end of the second rod 482 is connected to the second connecting end 4502. Therefore, the length of the bearing rod 48 with the mapping electrode 452 is greater than that of the bearing rod 48 without the mapping electrode 452, so that the area reduction of the first main mesh 371B, the first sub-mesh 391B and the second sub-mesh 392B corresponding to the first rods 481 and the area increase of the second main mesh 371C, the first sub-mesh 391C and the second sub-mesh 391C corresponding to the second rods 482 can cancel each other out, so that the surface area S_{cf} of the connecting frame 304 will not decrease due to the setting of the mapping electrode 452. In this way, the setting of the mapping electrode 452 can improve the mapping effect while ensuring that the ablation effect is not affected.

### Embodiment 6

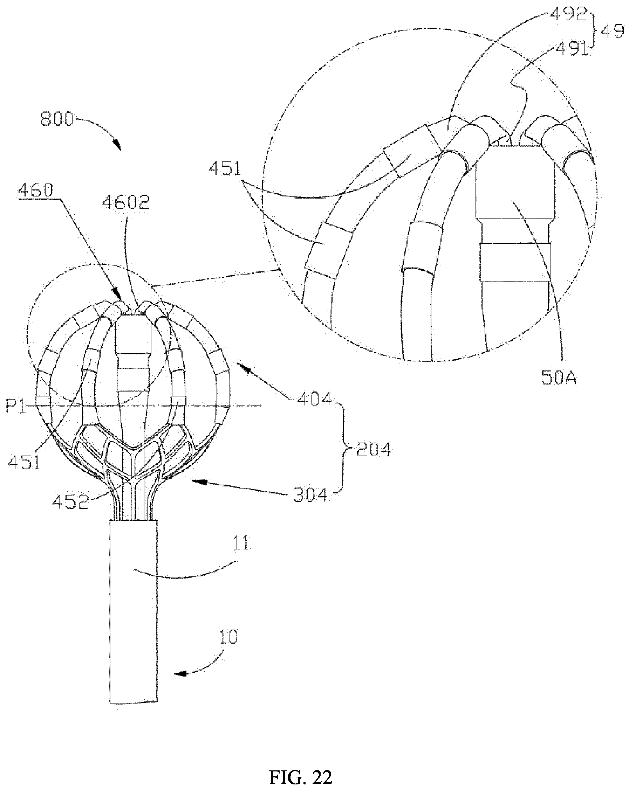

Please referring to Fig. 19 and Fig. 22, Fig. 22 is a schematic view of an ablation catheter 800 provided in a sixth embodiment of the present application. The support framework 204 includes a connecting frame 304 and a bearing frame 404 that are connected to each other in the axial direction. The proximal end of connecting frame 304 is connected to the distal end of catheter body 10, and the distal end of connecting frame 304 is connected to the proximal end of bearing frame 404. The bearing frame 404 includes multiple bearing rods 49. The difference between the ablation catheter 800 provided in the sixth embodiment of the present application and the ablation catheter 600 provided in the fourth embodiment is that: each bearing rod 49 includes a rod body 491 and an insulating sleeve 492 mounted around the rod body 491. It should be noted that the remaining structure of the ablation catheter 800 provided in the sixth embodiment is the same as that of the fourth embodiment, and will not be repeated here.

The distal end of each rod body 491 extends beyond the distal end of the insulating sleeve 492 and forms a bending portion 4602, which is fixed inside the traction member 50A. The distal ends of the multiple rods 491 are connected to the distal end of the traction member 50A. Multiple bending portions 4602 are arranged along the circumference of the support framework 204 to form a reverse portion 460. In this embodiment, the farthest end of the insulating sleeve 492 is located at the bending position of the bending portion 4602. In some embodiments, the farthest end of the insulating sleeve 492 is close to the bending position of the bending portion 4602, and located between the bending position of the bending portion 4602 and the proximal end of the rod body 491. As a result, the distal end of each insulating sleeve 492 does not exceed the bending position of the rod body 491, which is beneficial for the sheath retraction of the support framework 205, avoiding the ablation electrode 451 on the support framework 205 from scratching the inner wall of the delivery channel, and thus avoiding the risk of embolism.

For example, in this embodiment, the rod body 491 can be made of nickel titanium wire to provide excellent elastic properties and strength, enabling it to adhere well to the target tissue area. In some embodiments, the surface of the rod body 491 is provided with an insulating coating. For example, vacuum coating on the surface of nickel titanium wire to form an insulating coating. It can be understood that in some embodiments, the rod body 491 can also be made of other materials, such as stainless steel or polymer materials. The ablation electrode 451 and the mapping electrode 452 are set on the insulating sleeve 492, ensuring insulation between the ablation electrode 451 and the mapping electrode 452 and the rod body 491.

The material of the insulating sleeve 492 is PEBAX or other polymer insulation materials. The insulating sleeve 492 can be configured with one or more layers, which is not specifically limited in this application. In this embodiment, the insulating sleeve 492 is a single layer. The cross-sectional shape of the rod body 491 may be, but is not limited to, circular, semi-circular, shuttle shaped, or other shapes, which is not specifically limited in this application.

The above provides a detailed description to the embodiments of the present application. Specific embodiments are used in this disclosure to explain the principles and implementation methods of the present application. The above embodiments are only used to help understand the methods and core ideas of the present application, meanwhile, for those skilled in the art, there may be modifications in the specific implementation methods and application scope based on the ideas of this application. In summary, the content of this specification should not be understood as limiting this application.

## Claims

1. An ablation catheter, comprising:
a catheter body;
a support framework being provided at a distal end of the catheter body, comprising a connecting frame and a bearing frame being arranged distally relative to the connecting frame, a distal end of the connecting frame being connected to a proximal end of the bearing frame, a proximal end of the connecting frame being connected to the distal end of the catheter body, a conducting portion being provided on the connecting frame, an electrode unit insulated from the conducting portion being provided on the bearing frame, an electrode area of the electrode unit being less smaller than that of the conducting portion;
wherein, the conducting portion is capable of connecting one pole of an energy generator, and the electrode unit is capable of connecting another pole of the energy generator, so that ablation energy output by the energy generator is transferred to a target tissue region through a cooperation of the conducting portion and the electrode unit.

2. The ablation catheter according to claim 1, wherein the connecting frame comprises a plurality of connecting rods connected end-to-end to form a mesh structure, a proximal end of the mesh structure is connected to the distal end of the catheter body, and a distal end of the mesh structure is connected to the proximal end of the bearing frame.

3. The ablation catheter according to claim 2, wherein the mesh structure comprises a plurality of mesh units arranged circumferentially around a central axis of the support framework, a distal end of each mesh unit is connected to the proximal end of the bearing frame, and a proximal end of each mesh unit is connected to the distal end of the catheter body, and two adjacent mesh units are connected to each other in a circumferential direction of the support framework.

4. The ablation catheter according to claim 3, wherein the mesh structure further comprises a plurality of connecting meshes, in the circumferential direction of the support framework, two adjacent mesh units are connected by at least one connecting mesh; each mesh unit comprises at least one main mesh, the at least one connecting mesh shares one side with each of at least two main meshes of the two adjacent mesh units, and the main mesh and the connecting mesh each are configured with a polygonal-shaped profile.

5. The ablation catheter according to claim 4, wherein each mesh unit comprises two main meshes, namely a first main mesh and a second main mesh, the first main mesh and the second main mesh share one mesh node, two adjacent first main meshes share one mesh node, and two adjacent second main meshes share one mesh node;
a first connecting mesh and a second connecting mesh are provided between two adjacent mesh units, the first connecting mesh shares one side with each of two first main meshes and two second main meshes of the two adjacent mesh units, the second connecting mesh shares one side with each of two second main meshes of the two adjacent mesh units, two adjacent second connecting meshes shares one side with each other, the second connecting mesh is configured as an open polygon, and a proximal end of the second connecting mesh is connected to the distal end of the catheter body.

6. The ablation catheter according to claim 4, wherein each mesh unit comprises two main meshes, namely a first main mesh and a second main mesh, the first main mesh and the second main mesh share one mesh node, two adjacent second main meshes share one side with each other, the second main mesh is configured as an open polygon, and a proximal end of the second main mesh is connected to the distal end of the catheter body;
a connecting mesh is provided between two adjacent mesh units, and the connecting mesh shares one side with each of two first main meshes and two second main meshes of the two adjacent mesh units.

7. The ablation catheter according to claim 6, wherein the connecting mesh comprises a first sub-mesh, a second sub-mesh, and a third sub-mesh, the first sub-mesh, the second sub-mesh and the third sub-mesh share one mesh node, and the third sub-mesh shares one side with each of the first sub-mesh and the second sub-mesh;
the first sub-mesh shares one side with one of two first main meshes of the two adjacent mesh units, the second sub-mesh shares one side with another one of two first main meshes of the two adjacent mesh units, the first sub-mesh and the third sub-mesh together share one side with one of two second main meshes of the two adjacent mesh units, and the second sub-mesh and the third sub-mesh together shares one side with another one of two second main meshes of the two adjacent mesh units.

8. The ablation catheter according to claim 6, wherein the connecting mesh comprises a first sub-mesh and a second sub-mesh, the first sub-mesh and the second sub-mesh shares one side with each other, the first sub-mesh shares one side with each of the first main mesh and second main mesh of the same mesh unit, and the second sub-mesh shares one side with each of the first main mesh and second main mesh of the same mesh unit.

9. The ablation catheter according to any one of claims 1-8, wherein the bearing frame comprises a plurality of bearing rods arranged at intervals around a central axis of the support framework, distal ends of the plurality of bearing rods are close to the central axis of the support framework and fixed to each other, and proximal ends of the plurality of bearing rods are connected to the distal end of the connecting frame.

10. The ablation catheter according to claim 9, wherein the connecting frame as a whole is configured as the conducting portion, and the electrode unit comprises a plurality of electrodes arranged on the bearing rods and spaced from the conducting portion.

11. The ablation catheter according to claim 10, wherein the plurality of bearing rods comprise a plurality of first bearing rods and a plurality of second bearing rods, and all of the electrodes are arranged on the first bearing rods; at least two first bearing rods are arranged between two adjacent second bearing rods, and different numbers of electrodes are provided on the at least two first bearing rods.

12. The ablation catheter according to claim 11, wherein the plurality of electrodes comprise a plurality of first electrodes and a plurality of second electrodes, each of the first bearing rods is provided with the first electrode, the plurality of first electrodes form a first ring in a circumferential direction of the support framework, the plurality of second electrodes are provided on some of the plurality of first bearing rods and evenly spaced from each other in the circumferential direction, the plurality of second electrodes form a second ring in the circumferential direction of the support framework, the first ring is relatively close to the proximal end of the support framework, the second ring is relatively close to the distal end of the support framework, and a plane where the first ring is located and a plane where the second ring is located both are perpendicular to the central axis of the catheter body.

13. The ablation catheter according to claim 10, wherein, when only some of the bearing rods is in contact with the target tissue area, at least one electrode on the some of the bearing rods and the conducting portion are conducted to transfer the ablation energy to the target tissue area, and wherein the at least one electrode has a polarity different from that of the conducting portion.

14. The ablation catheter according to claim 10, wherein, when only some of the bearing rods is in contact with the target tissue area, electrodes with opposite polarities on the some of the bearing rods are conducted to transfer the ablation energy to the target tissue area.

15. The ablation catheter according to claim 14, wherein the electrodes with opposite polarities are all located on the same bearing rod; or, the electrodes with opposite polarities are located on multiple bearing rods which are spaced from each other.

16. The ablation catheter according to claim 1, wherein the ablation catheter further comprises a microelectrode for detecting electrocardiogram signals, the microelectrode is set on the farthest end of the connecting frame and insulated from the conducting portion and the electrode unit.

17. The ablation catheter according to claim 16, wherein the connecting frame is provided with a mounting hole for installing the microelectrode.

18. The ablation catheter according to any one of claims 1-10, wherein the ablation catheter further comprises a traction member, which is movably arranged in the catheter body and extends beyond the distal end of the catheter body, and distal ends of the plurality of bearing rods are gathered and connected to a distal end of the traction member.

19. The ablation catheter according to claim 18, wherein the ablation catheter further comprises a reference electrode set on at least one of the catheter body and the traction member, and the reference electrode is configured for electrocardiogram signal mapping to obtain a reference potential.

20. The ablation catheter according to claim 18, wherein the ablation catheter further comprises a head electrode set on the farthest end of the traction member, and the head electrode is configured to cooperate with the conducting portion and the plurality of electrodes to release ablation energy, as well as for electrocardiogram signal mapping.

21. The ablation catheter according to claim 20, wherein the distal end of the bearing frame and the distal end of the head electrode are arc-shaped and smoothly connected.

22. The ablation catheter according to claim 18, wherein the distal end of the bearing frame is provided with a reverse portion, a proximal end of the reverse portion is connected to the traction member, and wherein the farthest ends of the bearing rods bend towards the proximal end and the central axis of the support framework gradually to form the reverse portion.

23. The ablation catheter according to claim 22, wherein the ablation catheter further comprises a connecting member, and the reverse portion is connected to the traction member through the connecting member.

24. The ablation catheter according to claim 23, wherein the connecting member comprises a first connecting portion connected to the reverse portion and a second connecting portion connected to the first connecting portion and the traction member, a distal end of the first connecting portion is provided with a plurality of positioning holes extending axially, and the plurality of bearing rods are fixed in the plurality of positioning holes, respectively.

25. The ablation catheter according to claim 24, wherein the second connecting portion is inserted into the traction member, and the first connecting portion is blocked by the traction member.

26. The ablation catheter according to claim 18, wherein the support framework has an equatorial position located between its proximal end and distal end, and the plurality of electrodes comprises multiple ablation electrodes located between the equatorial position of the support framework and the distal end of the bearing frame.

27. The ablation catheter according to claim 26, wherein the ablation catheter further comprises at least one mapping electrode provided on the bearing frame, the at least one mapping electrode is located on the equatorial position or located between the equatorial position and the proximal end of the bearing frame, and wherein the mapping electrode is configured for detecting electrophysiological signals of the target tissue region.

28. The ablation catheter according to claim 27, wherein the plurality of bearing rods comprise multiple first rods and multiple second rods, and the multiple first rods and multiple second rods are alternately arranged around the central axis of the support framework, the at least one mapping electrode comprises multiple mapping electrodes, and each first rod is provided with at least one of the multiple mapping electrodes.

29. The ablation catheter according to claim 28, wherein an end of the connecting frame facing the bearing frame comprises a first connecting end and a second connecting end, the first connecting end is closer to the proximal end of the connecting frame than the second connecting end, a length of the first rod is greater than that of the second rod, a proximal end of the first rod is connected to the first connecting end, and a proximal end of the second rod is connected to the second connecting end.

30. The ablation catheter according to any one of claims 26-29, wherein the bearing rod comprises a rod body and an insulating sleeve mounted around on the rod body, a distal end of the rod body extends beyond a distal end of the insulating sleeve and forms a bending portion, a distal end of the bending portion is fixed inside the traction member, and wherein the farthest end of the insulating sleeve is located at a bending position of the bending portion, or, the farthest end of the insulating sleeve is adjacent to the bending position of the bending portion, and the insulating sleeve is positioned between the bending position of the bending portion and the proximal end of the rod body.
